# EUROPEAN PATENT SPECIFICATION

(11) **EP 0 870 057 B1**
(45) Date of publication and mention of the grant of the patent: **24.09.2003**
(21) Application number: 96942779.8
(22) Date of filing: 15.11.1996
(51) Int. Cl.: C12Q 1/68

(54) **METHOD FOR IDENTIFYING METASTATIC SEQUENCES**
VERFAHREN ZUR IDENTIFIZIERUNG METASTATISCHER SEQUENZEN
PROCEDE D'IDENTIFICATION DE SEQUENCES METASTASIQUES

(30) Priority: 16.11.1995 US 6838; 30.01.1996 US 594031
(43) Date of publication of application: 14.10.1998
(73) Proprietor: THE BAYLOR COLLEGE OF MEDICINE, Houston, TX 77030-3498 (US)
(72) Inventor: THOMPSON, Timothy, Houston, TX 77096 (US)
(74) Representative: Harrison Goddard Foote
(86) International application number: US9618567
(87) International publication number: WO97018454

(56) References cited:
- WO-A-86/03226
- WO-A-94/28129
- WO-A-95/19369
- WO-A-96/30389
- SCIENCE, vol. 257, no. 5072, 14 August 1992, pages 967-971, XP000508268 LIANG P ET AL: "DIFFERENTIAL DISPLAY OF EUKARYOTIC MESSENGER RNA BY MEANS OF THE POLYMERASE CHAIN REACTION"
- THE JOURNAL OF HISTOCHEMISTRY AND CYTOCHEMISTRY, vol. 42, no. 4, 1 April 1994, pages 505-511, XP000575423 WOOD D P ET AL: "SENSITIVITY OF IMMUNOHISTOCHEMISTRY AND POLYMERASE CHAIN REACTION IN DETECTING PROSTATE CANCER CELLS IN BONE MARROW"
- CANCER RESEARCH, vol. 52, December 1992, pages 6966-6968, XP002032010 LIANG ET AL.: "Differential display and cloning of messenger RNAs from human breast cancer versus mammary epthelial cells"
- PROCEEDINGS OF THE ANNUAL MEETING OF THE AMERICAN ASSOCIATION FOR CANCER RESEARCH, TORONTO, MAR. 18 - 22, 1995, vol. 36, 18 March 1995, AMERICAN ASSOCIATION FOR CANCER RESEARCH, page 266 XP002019344 ROBSON C N ET AL: "IDENTIFICATION OF PROSTATIC ANDROGEN REGULATED GENES USING THE DIFFERENTIAL DISPLAY TECHNIQUE"
- ONKOLOGIE, vol. 18, no. SUPPL. 03, 1 November 1995, pages 2-7, XP000576595 SCHLAG P M ET AL: "FRUEHERKENNUNG VON KREBS MIT HILFE VON MOLEKULARBIOLOGISCHEN MARKERN"
- PNAS, vol. 87, October 1990, pages 7678-7682, XP002031777 WELCH ET AL.: "Transforming growth factor beta stimulates mammary adenocarcinoma cell invasion and metastatic potential"
- CELL, vol. 56, 1989, pages 917-930, XP002031778 THOMPSON ET AL.: "Multistage carcinogenesis induced by ras and myc oncogenes in a reconstituted organ"
- FORTH,HENSCHLER,RUMMEL: "Allgemeine und spezielle Pharmakologie und Toxikologie" 1987 , WISSENSCHAFTSVERLAG,MANNHEIM,DE XP002031779 149990 see page 716 - page 723

## Description

### Background

### 1. Field of the Invention

The present invention relates to methods for the identification and isolation of metastatic sequences, to diagnostic probes and kits which contain metastatic sequences and to therapeutic treatments for neoplastic disorders based on metastatic sequences.

### 2. Description of the Background

The development of higher organisms is characterized by an exquisite pattern of temporal and spatially regulated cell division. Disruptions in the normal physiology of cell division are almost invariably detrimental. One such type of disruption is cancer, a disease that can arise from a series of genetic events.

Cancer cells are defined by two heritable properties, uncontrolled growth and uncontrolled invasion of normal tissue. A cancerous cell can divide in defiance of the normal growth constraints in a cell leading to a localized growth or tumor. In addition, some cancer cells also gain the ability to migrate away from their initial site and invade other healthy tissues in a patient. It is the combination of these two features that make a cancer cell especially dangerous.

An isolated abnormal cell population that grows uncontrollably will give rise to a tumor or neoplasm. As long as the neoplasm remains in a single location, it is said to be benign, and a complete cure may be expected by removing the mass surgically. A tumor or neoplasm is counted as a cancer if it is malignant, that is, if its cells have the ability to invade surrounding tissue. True malignancy begins when the cells cross the basal lamina and begin to invade the underlying connective tissue. Malignancy occurs when the cells gain the ability to detach from the main tumor mass, enter the bloodstream or lymphatic vessels, and form secondary tumors or metastases at other sites in the body. The more widely a tumor metastasis, the harder it is to eradicate and treat.

As determined from the epidemiological and clinical studies, most cancers develop in slow stages from mildly benign into malignant neoplasms. Malignant cancer usually begins as a benign localized cell population with abnormal growth characteristic called a dysplasia. The abnormal cells acquire abnormal growth characteristics resulting in a neoplasia characterized as a cell population of localized growth and swelling. If untreated, the neoplasia *in situ* may progress into a malignant neoplasia. Several years, or tens of years may elapse from the first sign of dysplasia to the onset of full blown malignant cancer. This characteristic process is observed in a number of cancers. Prostate cancer provides one of the more clear examples of the progression of normal tissue to benign neoplasm to malignant neoplasm.

The walnut-sized prostate is an encapsulated organ of the mammalian male urogenital system. Located at the base of the bladder, the prostate is partitioned into zones referred to as the central, peripheral and transitional zones, all of which surround the urethra. Histologically, the prostate is a highly microvascularized gland comprising fairly large glandular spaces lined with epithelium which, along with the seminal vesicles, supply the majority of fluid to the male ejaculate. As an endocrine-dependent organ, the prostate responds to both the major male hormone, testosterone, and the major female hormones, estrogen and progesterone. Testicular androgen is considered important for prostate growth and development because, in both humans and other animals, castration leads to prostate atrophy and, in most cases, an absence of any incidence of prostatic carcinoma.

The major neoplastic disorders of the prostate are benign enlargement of the prostate, also called benign prostatic hyperplasia (BPH), and prostatic carcinoma; a type of neoplasia. BPH is very common in men over the age of 50. It is characterized by the presence of a number of large distinct nodules in the periurethral area of the prostate. Although benign and not malignant, these nodules can produce obstruction of the urethra causing nocturia, hesitancy to void, and difficulty in starting and stopping a urine stream upon voiding the bladder. Left untreated, a percentage of these prostate hyperplasia and neoplasias may develop into malignant prostate carcinoma.

In its more aggressive form, transformed prostatic tissues escape from the prostate capsule and metastasize invading locally and throughout the bloodstream and lymphatic system. Metastasis, defined as tumor implants which are discontinuous with the primary tumor, can occur through direct seeding, lymphatic spread and hematogenous spread. All three routes have been found to occur with prostatic carcinoma. Local invasions typically involve the seminal vesicles, the base of the urinary bladder, and the urethra. Direct seeding occurs when a malignant neoplasm penetrates a natural open field such as the peritoneal, pleural or pericardial cavities. Cells seed along the surfaces of various organs and tissues within the cavity or can simply fill the cavity spaces. Hematogenous spread is typical of sarcomas and carcinomas. Hematogenous spread of prostatic carcinoma occurs primarily to the bones, but can include massive visceral invasion as well. It has been estimated that about 60% of newly diagnosed prostate cancer patients will have metastases at the time of initial diagnosis.

Surgery or radiotherapy is the treatment of choice for early prostatic neoplasia. Surgery involves complete removal of the entire prostate (radical prostatectomy), and often removal of the surrounding lymph nodes, lymphadenectomy. Radiotherapy, occasionally used as adjuvant therapy, may be either external or interstitial using ¹²⁵I. Endocrine therapy is the treatment of choice for more advanced forms. The aim of this therapy is to deprive the prostate cells, and presumably the transformed prostate cells as well, of testosterone. This is accomplished by orchiectomy (castration) or administration of estrogens or synthetic hormones which are agonists of luteinizing hormone-releasing hormone. These cellular messengers directly inhibit testicular and organ synthesis and suppress luteinizing hormone secretion which in turn leads to reduced testosterone secretion by the testes. Despite the advances made in achieving a pharmacologic orchiectomy, the survival rates for those with late stage carcinomas are rather bleak.

### Summary of the Invention

The present invention overcomes the problems and disadvantages associated with current strategies and designs and provides new methods for the identification of sequences related to metastasis.

The invention is directed to methods for the identification of a metastatic sequence as defined in claim 1. One or more oncogenic sequences are transfected into a cell to form a transfected cell. The transfected cell is introduced into a primary site of a host animal to establish a colony which is incubated in the animal for a period of time sufficient to develop both a primary tumor and a malignant tumor. Expressed sequences are harvested from the primary tumor and the metastasis. Harvested sequences are compared to each other and to non-metastatic cells to identify sequences related to metastasis. Dominant metastatic genes are genes whose expression leads to metastasis. Such genes are typically expressed at high levels in metastatic cells and not significantly expressed in normal or nonmetastatic cells. Recessive metastatic genes, genes whose expression prevents metastasis, may be selectively expressed in normal and nonmetastatic cells and absent in metastatic cells. Dominant and recessive metastatic genes may act directly or act pleiotropically by enhancing or inhibiting the expression or function of other dominant and recessive metastatic genes.

Another embodiment of the invention is directed to methods for identifying metastatic sequences as defined in claim 24. A mammalian cell is treated with a metastatic agent and the treated cell is implanted into a primary site of a host mammal. The host animal is maintained for a period of time sufficient for the cells to proliferate and to develop a metastatsis at a secondary site. Expressed sequences from cells of the primary site and cells of the secondary site are reverse transcribed into cDNA by differential display polymerase chain reaction to identify differentially expressed sequences.

Other objects and advantages of the invention are set forth in part in the description which follows, and in part, will be obvious from this description, or may be learned from the practice of the invention.

### Description of the Drawings

- Figure 1: Schematic showing two paths in the multistep progression to cancer.
- Figure 2: Staining of primary tumor (A) and metastatic deposit (B) from the lung of the same animal
- Figure 3: Staining of normal human prostate (A), moderately differentiated human prostate tumor (B and C), and poorly differentiated prostate tumor (D).
- Figure 4: Schematic of method for isolating a metastatic gene from a gene ablated mouse strain.
- Figure 5: Schematic showing method to establish a tumor and a metastatic transplant from fetal tissue(A) and from cell lines and tumors (b).
- Figure 6: Isolation and characterization of nmb gene expression by DD-PCR and RNA blot in primary and metastatic cells.
- Figure 7: Differential expression of multiple genes is determined by DD-PCR and RNA blot of primary and metastatic cells.
- Figure 8: Caveolin identified as a differentially expressed gene by DD-PCR.
- Figure 9: Differential expression of genes isolated by DD-PCR confirmed by RNA blots.
- Figure 10: RNA blot analysis of total tumor mRNA using clone 29 GADPH probes.
- Figure 11: RNA blot of three independent MPR metastatic tumors and 5 MPR non-metastatic tumors.
- Figure 12: Nucleotide sequences of metastatic nucleic acids.
- Figure 13: Characterization of metastatic sequences isolated.
- Figure 14: Immunohistological staining of primary and metastatic human prostate tumors using anti-caveolin antibodies.

### Description of the Invention

As embodied and broadly described herein, the present invention is directed to methods for identifying metastatic sequences.

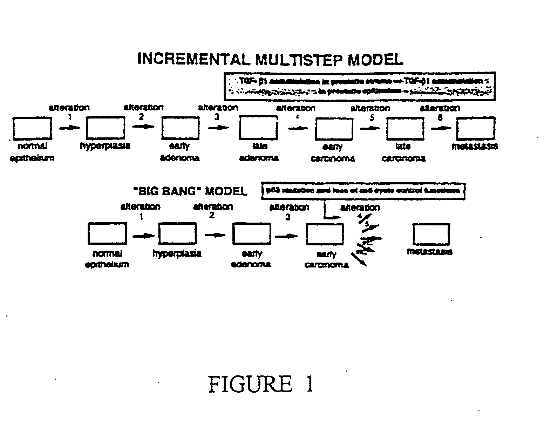

The ability of cancers to metastasize makes tumors difficult to eradicate by any means. Malignant cancer involves a multistage progression from, for example, normal tissue through hyperplasia, early adenoma, early carcinoma and finally to a metastatic tumor (Figure 1). Cells of a typical tumor loosen their adhesion to their original cellular neighbors and cross the basal lamina and endothelial lining to enter the body's circulation. Once in circulation, the metastatic cell exits from the circulation to disseminate throughout body and proliferate in a new environment.

Like the initial oncogenic event, the ability of a cell to metastasize requires additional mutationic or epigenetic changes. An understanding of the molecular mechanisms of metastasis allow for the design of treatments to inhibit metastasis. Knowledge of stage specific gene expression for neoplastic disorders allows for early detection and typing of tumors. With early detection and typing, proper treatment may be administered to a patient with the neoplastic disorder earlier, which will lead to a higher probability of a complete cure.

For human prostate tumors, the study of stage specific tumors is difficult, if not impossible, as cell lines are extremely difficult to grow and it is rare that tissue becomes available from the primary tumor as well as metastatic disease from the same patient This problem is exacerbated because of the infrequent biopsy of metastatic deposits in concordance of isolation of material from the primary tumor. Furthermore, the growth of cell lines from malignant prostates has proved to be problematic over the last few decades. This is evidenced by the lack of cell lines from prostate cancer obtained under any conditions.

One embodiment of the invention is directed to a method for identifying a metastatic sequence. A mammalian cell is transformed into a pre-neoplastic or neoplastic state or phenotype by transfection with one or more oncogenic sequences. Alternatively, or in addition to transfection, the mammalian cell may be treated with an agent or subjected to a condition that potentiates the metastatic character of the cell or predisposes the cell to metastasis. The transfected or treated cell is implanted into a host animal at a primary site and grown for a period of time sufficient to develop a metastasis at a secondary site. Expressed sequences from cells of the primary site and cells at the secondary site are amplified by differential display polymerase chain reactions. PCR products from these reactions are compared and the metastatic sequence identified by alteration in the levels or patterns of the resulting products.

Mammalian cells from a wide variety of tissue types and species are suitable for transfection or treatment including surgically obtained or primary or immortalized cells and cell lines. Cells may be from humans or primates, mice, rats, sheep, cows, rabbits, horses, pigs or guinea pigs or from transgenic or xenogeneic host mammals. Cells may be obtained from adult, juvenile or fetal tissue, and used directly from the mammal, from cryogenically preserved samples, or after culturing *in vitro* or *in vivo* for a period of time. *In vitro* culturing typically involves tissue culture conditions (*e.g*. 37°C; 5% CO₂) while *in vivo* culturing may involve successive passage of cells through host animals such as, for example, mice or rabbits. Cells passed *in vivo* may be obtained from sites proximal or distal to the site of implantation. The tissue type from which the cells are derived or obtained may be any tissue which is susceptible to transfection or other treatment including, for example, urogenital tissues, epithelial cells, hepatic cells, fibroblasts lymphatic tissues, hematopoietic cells, cells of the immune system, cells of the gastrointestinal system and cells of the nervous system.

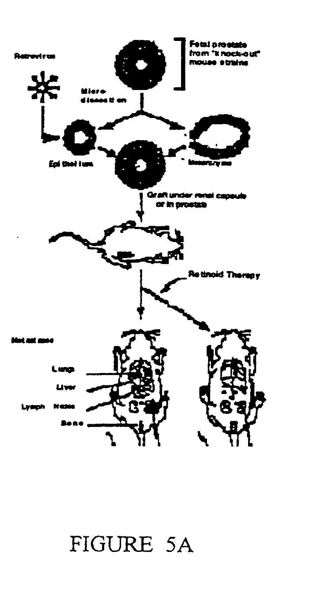

Cell types useful for the identification of metastatic sequences related to prostrate cancer include cells and cell lines of the fetal prostate lineage from normal or transgenic animals, and cells from normal or reconstituted prostate tissue. One method of generating reconstituted prostate cells is to isolate fetal prostate tissue and microdissect the fetal prostate epithelium away from fetal mesenchyme. Fetal prostate epitheliums may be genetically manipulated before reassociation with fetal mesenchyme (Figure 5A). Genetic manipulation involves treatment or transfection with a metastatic agent or a nucleic acid sequence that affects neoplastic or metastatic potential of the cell. Reassociation of fetal epithelium and mesenchyme is performed by implanting epithelium tissue within a pocket of mesenchyme tissue. After manipulation, cells are reimplanted into a mammalian host in a similar manner as other cells, such as reimplantation into or under the renal capsule.

Mammalian cells may be transfected by a variety of techniques, all of which are well-known to those of ordinary skill. Direct methods involve the introduction of genetic material into the nucleus of a cell by injection. These techniques include high velocity projectile injection, microinjection, and electroporation. Indirect methods, involving the active or passive uptake of the genetic information by the cell. Indirect techniques include transduction with recombinant vectors, and chemical or physical treatments such as calcium phosphate uptake, lipofection or dextran sulfate transfection. Chemical techniques rely on chemical carriers to introduce nucleic acids into a cell. These methods, for example, utilize unilamellar phospholipid vesicles (*e.g*. liposomes) loaded with DNA (or RNA). The approach relies on the fusion of the DNA containing vesicles with the plasma membrane of the recipient cells. After entry, DNA traverse the cytoplasm and enter the nucleus. Another lipofection technique uses a synthetic cationic lipid such as N-[1-(2,3-dioleyloxy)propyl]-N,N,N-trimethylammonium chloride (DOTMA). DOTMA spontaneously associates with nucleic acids and forms unilamellar vesicles upon sonication. Genetic material is incorporated into these vesicles and subsequently transfected into the cell. Calcium phosphate co-precipitation involves mixing of purified nucleic acid with buffers containing phosphate and calcium chloride which results in the formation of a fine precipitate. Presentation of this precipitate to cells results in incorporation of the nucleic acid into cellular genome. Other chemicals, such as DEAE dextran or polybrene, when present in media with nucleic acids, can also cause the transfection of mammalian cells.

Physical methods of transfection rely on electric fields, needles and particles to enable nucleic acids to traverse the cellular membrane. Electric field mediated DNA transfection, commonly called electroporation, is based on the principle that membranes, when subjected to an electric field, undergo a reversible breakdown resulting in pores large enough to permit the passage of nucleic acids. In micro-projectile mediated gene transfer, microprojectiles of subcellular dimensions are coated with nucleic acid and propelled at high velocity into a cell using a particle gun. The nucleic acid is introduced into the nucleus directly when the particles impinge upon the nucleus. In nucroinjection, nucleic acid is injected directly into the nucleus of a cell with a needle. Lasers have also been used to introduce minute holes in cellular membrane to allow introduction of nucleic acids. All these methods may be used for transfection and the selection of the method will depend on the cell type, the desired transfection efficiency and the equipment available.

The efficiency of transfection may be monitored and enhanced by the co-transfection of a selectable marker. If a marker is co-transfected with a genetic construct, positively transformed cells may be separated from nontransformed cells by chemical selection. The efficiency of transfection will be increased in most cases because the chemicals will selectively kill non-transfected cells. The number of transfected cells may also be monitored by analyzing the degree of chemical resistance of the transfected cells. Markers commonly used for selection purposes include, for example, nucleic acids encoding dihydrofolate reductase, metallothionein, CAD, adenosine deaminase, adenylate deaminase, UMP synthetase, IMP 5'-dehydrogenase, xanthine-guanine phosphoribosyltransferase, mutant thymidine kinase, mutant HGPRTase, thymidylate synthetase, P-glycoprotein 170, ribonucleotide reductase, glutamine synthetase, asparagine synthetase, arginosuccinate synthetase, ornithine decarboxylase, HMG-CoA reductase, N-acetylglucosaminyl transferase, theronyl-tRNA synthetase, sodium or potassium dependent ATPase or derivatives or mutants of these nucleic acids. Markers may be used individually or in combination. Chemicals useful for selection include methotrexate, cadmium, PALA, Xyl-A, adenosine, 2'-deoxycoformycin, adenine, azaserine, coformycin, 6-azauridine, pyrazofuran, mycophenolic acid, limiting xanthine, hypoxanthine, aminopterin, thymidine, 5-fluorodeoxyuridine, adriamycin, vincristine, colchicine, actinomycin D, puromycin, cytocholasin B, emetine, maytansine, Bakers' antifolate, aphidicolin, methionine sulfoximine, β-aspartyl hydroxamate, albizziin, canavanine, α-difluoromethylornithine, compactin, tunicamycin, borrelidin, ouabain, and derivatives and analogs and combinations of these chemicals. Some chemicals, such as methotrexate, may be used individually while other chemicals, such as HAT (hypoxanthine, aminopterin and thymidine), need to be used in combination to be effective.

The oncogene transfection efficiency, the fraction of live cells transfected by an oncogene, may be indirectly enhanced by chemical selection for a co-transfected marker. An oncogene is a sequence which can predispose, or induce the cell into a pre-neoplastic or neoplastic condition or otherwise enhance the metastatic potential of the cell. Sequences with these properties are referred to as oncogenes and include *abl, ahi, akt, bcl, crk, dsi, erb, ets, evi, fes*/*fps, fim, fis, fgr, flv, fms, fos, gin, gli, int, jun, kit, mas, lck, met, mil*/*raf, mis, mlv, mos, myb, myc, neu, onc, pim, raf, ras, rel, ros, seq, sis, ski, spi, src, tcl, thy, trk,* and *yes*. Some oncogenes, such as *ras*, are oncogenic when mutated. Other oncogenes, such as *myc*, are oncogenic when overexpressed or underexpressed. Many oncogenes represent members of multigene families or homologs families. Homologs are proteins that have similar primary, secondary or tertiary structures. Genes may differ in nucleic acid sequence or encoded peptide sequence and still be homologs when the encoded polypeptides have similar spatial folding. Many oncogenes can be classified into dominant oncogenes and recessive oncogenes. One or more dominant oncogenes can confer a neoplastic or preneoplastic phenotype to a cell. One or more recessive oncogenes, when silenced, may also confer a neoplastic or preneoplastic phenotype. Gene silencing is performed by transfecting cells with nucleic acids which cause genetic ablation or by antisense suppression.

While any oncogene may be used, the preferred oncogenes are those that are normally associated with metastasis such as a metastasis specific gene. Such genes include for example, *TGF-β1, Cyclin D1 p21*, *p34, mutant p53, lysyl oxidase, caveolin, actin binding protein, ubiquitin activating enzyme E1, nmb or α-actinin 3.* Metastatic-specific genes may be used individually or in combination with other oncogenes.

The metastatic potential of a cell may be altered, for example, by gene ablation with a sequence specific for a recessive oncogene. Recessive oncogene are those genes which encode products which can suppress oncogenesis and metastasis. A gene ablation sequence can be designed to specifically suppress a recessive oncogene. Ablation may include pre-transcriptional inhibition such as homologous recombination with endogenous recessive oncogenes and post transcriptional inhibition such as the expression of antisense oncogenes to suppress translation. Gene ablation sequences may be targeted towards well known recessive oncogenes such as, for example, the retinoblastoma gene (Rb) or Bcg. Other candidates for ablation include metastatic genes previously isolated by the invention such as, for example, TGF-β1, cyclin D1, p21, p34, mutant p53, lysyl oxidase, caveolin, actin binding protein, ubiquitin activating enzyme E1, nmb or α-actinin-3. The effects of ablating a recessive oncogene may include oncogenesis and metastases.

Alternatively, or in addition to transfecting the mammalian cell may be treated with an agent, either before or after transfection, that alters the expression of the cell's nucleic acids. Treatment may comprise contacting the cells with one or more agents which affect the neoplastic (*e.g*. neoplastic agents; phorbol esters), metabolization (*e.g.* metabolic agents), metastatic (*e.g*. metastatic agents), differentiation (*e.g*. differentiation agents; retinoic acid), activation or proliferation (*e.g*. growth factors) of the cell. Agents which can alter gene expression include chemicals such as benzanthracene (BA), dimethyl benzanthracene (DMBA) or 5-azacytidine. Alternatively, treatment may also comprise altered conditions such as hypoxia which involves subjecting a cell to a reduced oxygen content, exposable to radiation or other stresses to the cell.

Treatment may be *in vitro* or *in vivo* and may include for example, direct or indirect induction or suppression of well know oncogenic sequences and genes isolated by the invention such as, for example, *TGF-β1*, *Cyclin D1,* mutant *p53, lysyl oxidase, caveolin, actin binding protein, ubiquitin activating enzyme E1, nmb, α actinin 3, and p34*. Gene expression induction includes transfecting expression vectors encompassing coding regions of the gene. Gene repression comprises introducing a gene ablation sequence or a repressor of the gene to the cell.

Cells winch have one or more genes ablated may also be used For example, a metastatic suppressor gene may be ablated to prevent inhibition to metastases. A useful gene for ablation is a gene capable of affecting the phenotype and behavior of a cell or tumor. For example, with prostate tumors, suitable genes include both well known genes and genes isolated by the methods of the invention such as for example, *TGF-β1*, *Cyclin D1, p21, p34, mutant p53, lysyl oxidase, caveolin, actin binding protein, ubiquitin activating enzyme E1, nmb and α actinin 3*. Genetic ablation (gene knockout) refers to a process of silencing the expression of a particular gene in a cell. The silencing process may include, for example, gene targeting or antisense blocking. Gene targeting refers to a process of introducing a nucleic acid construct into a cell to specifically recombine with a target gene. The nucleic acid construct inactivates the targeted gene. Inactivation may be by introduction of termination codons into a coding region or introduction of a repression site into a regulatory sequence. Antisense blocking refers to the incorporation into a cell of expression sequences which directs the synthesis of antisense RNA to block expression of a target gene. Antisense RNA hybridizes to the mRNA of the target gene to inhibit expression.

The host animal is preferably the same species as the implanted cell. In cases of xenogeneic transplants, the host may be immunocompromised genetically or by treatment with drugs such as immunosuppressants. A host may be immunocompromised genetically by breeding such as with nude mice or severe combined immunodeficient (SCID) mice. A host may also be immunocompromised by chemical or irradiation methods. An additional route to immunocompromise a host is to use transgenic technology to introduce an immunosuppressing gene or to introduce a foreign antigen gene. An immunosuppressing gene is a gene that affects the efficiency of the immune system such as a gene which inhibits the formation of cells of the B cell or T cell lineage. A foreign antigen gene, when expressed, may cause the host to tolerate the antigens in a xenogeneic transplant and not mount an immune response.

Cells may be implanted into any primary site in a host animal, such as, for example, subcutaneous implantation, intravenous injection, or implantation into the abdominal cardiac, chest, pulmonary, thoracic or peritoneal cavity. Using techniques known to those of ordinary skill in the art, cells can be placed on or in nearly any organ or tissue. Reasons for choosing a site include ease of implant, proximity of similar tissue type, immunoprivileged position and ease of inspection. Metastasises migrate from the primary site to one or more secondary sites such as, for example, the lung, kidney, liver, lymph nodes, brain, testis, bone, spleen, ovaries or mammary. Preferred sites include the renal capsule, the testes, the prostate and the ovaries.

To avoid histocompatibility problems, the implant may be placed into a histocompatible host animal. Such problems are generally avoided if the host animal are syngeneic. Alternatively, a non-histocompatible host may be used if the host can be made immunotolerant. Hosts may also be transgenic or immunocompromised animals or genetically matched to the mammalian cells to be introduced. Immunocompromised animals may be derived from established mouse lines such as nude mice or severe combined immune deficiency (SCID) mice, or by treatments such as radiation, chemical, pharmaceutical or genetic targeting. Sufficiently immunosuppressed animals can be made tolerant to xenogeneic transplants.

After implantation the host animal is maintained under normal conditions to develop metastases. Alternatively, the host animal may be subjected to an altered treatment or environmental condition to stimulate or repress metastasis or induce other cellular functions. In metastasis, a subpopulation of cells of the implantation site invade and establish one or more secondary colonies in the host animal. The behavior of the implanted cell will depend on the cell type, the transfected sequence and the implantation location. Typical secondary sites for metastatic colonies include lung, kidney, liver, lymph nodes, brain, testis, spleen, bone, ovary, skin and mammary tissue. Metastatic development times vary from days to weeks even months. Cells with a high metastatic potential tend to progress to metastasis quickly while cells with a low metastatic potential may require very long periods of time that span significant portions of the lifespan of the animal.

The host animal may be analyzed for metastatic development weekly, from one week to 20 weeks to six months, nine months or one year after implantation. For animals with longer lifespans such as sheep, the animal may be inspected yearly from one year on up to ten years for metastatic tumors. Metastases can be detected by examinations such as palpitation, biopsy, imaging, exploratory surgery, CAT scans, autopsy, X-ray and direct observation. In addition, tissue samples may be taken surgically from the host mammal and subjected to histological or other examination for the detection of metastases.

Expressed sequences include mRNA, rRNA, hnRNA, DNA, cDNA and any nucleic acid sequence that is expressed in the cell. These sequences may be amplified by *in situ* techniques or by purification of nucleic acid from collected cells. Expressed sequences may be obtained by extracting nucleic acids from cells before implantation, at the primary site or at the secondary site. Cells collected at these sites may optionally be cultured for a time before nucleic acid extraction. The effects of treatment with gene expression modifying agents or environmental conditions can be ascertained by collecting cells before and after treatment. Treatment may be applied to the cells while the cells are in the host mammal or after the cells are excised and in culture. Nucleic acid are collected from cells using techniques that are well known to those of ordinary skill in the art.

Expressed sequences may be used directly for polymerase chain reaction (PCR) analysis using, for example, the technique of reverse transcriptase polymerase chain reaction (RT-PCR). Alternatively, RNA may be enriched for mRNA using a poly-A RNA enrichment method. Numerous poly-A RNA enrichment methods exist and are commercially available. Techniques used for poly-A RNA enrichment include oligo-dT columns, oligo-dT magnetic beads, and oligo-dT cellulose. RNA may be further processed into cDNA before analysis by reverse transcription using reverse transcriptase. The cells or the extracted nucleic acid may be preserved, such as by freezing, and analyzed at a later time.

Differential display polymerase chain reactions (DD-PCR) are performed on the expressed sequences using two variable primers which may contain the same or entirely different sequences or an anchor primer and a variable primer. If an anchor primer is used, one anchor primer and one variable primer create a single or a single set of reaction products for each reaction. A complete profile may include 25 or more different PCR reactions per sample wherein each PCR reaction is performed with the same anchor primer and a different variable primer. DD-PCR may also be performed using anchor and variable primers which contain the same sequence. Whether a particular reaction is used depends on whether a difference exists between the products of two PCR reactions using the same primers. When a significant difference exists between the expression sequences amplified, one pair of PCR reactions may be sufficient and informative.

Anchor primers are preferably oligonucleotides with a poly-T sequence at the 5' -terminas and a dinucleotide selected from the group consisting of AA, AG, AC, AT, GA, GG, GC, GT, CA, CG, CC and CT at the 3'-terminas. For example, the sequence may be 5'-TTTTTTAA-3' or 5'-TTTTTTAG-3'. The length of the poly-T sequence is typically between about 5 to about 30 bases in length and preferably between about 10 to about 20 nucleotides long. The total length of the anchor primer can vary greatly for each experiment but is preferably between about 7 to about 32 and more preferably between about 12 and about 22. Differential diagnostic polymerase chain reaction may also be performed using an anchor primer of any sequence and a length between about 5 to about 30, preferably between about 5 to about 20 and more preferably between about 7 to about 12 bases.

The variable primer may comprise a random sequence, or a specific sequence such as, for example, a sequence of SEQ ID NO. 1 to SEQ ID NO. 24. Variable primers preferably are oligonucleotides with a length between about 5 to about 30, preferably between about 5 to about 20, and more preferably between about 7 to about 12 bases in length.

To enhance detection of the PCR product, the anchor primer or the variable primer, or both, may comprise a detectable moiety. Examples of detectable moieties include radioactive moieties, phosphorescent moieties, magnetic moieties, luminescent moieties, conjugatable moieties or other detectable moiety. A plurality of detectable moieties may be used to enhance detection or to simplify data analysis. Other detectable moieties include conjugatable moieties and molecules which can bind specifically to other molecules which are themselves detectable. Examples of conjugatable moieties include avidin, streptavidin, biotin, antibody, antigen, cell adhesion molecules and other molecules with similar activities. Detectable moieties are preferably labeled nucleotides. A nucleotide may be any natural or synthetic nucleotide or nucleotide analog capable of incorporation into an elongation reaction in a polymerase chain reaction. Labeled nucleotides include nucleotide triphosphates labeled with one or more radioactive atoms such as ³²P, ³³P, ³H, ¹⁴C and ³⁵S.

Products of DD-PCR reactions are compared to detect the metastatic sequence. Comparisons can be performed between expressed sequences from cells at secondary sites with cells at any stage in the method including untreated mammalian cells, transfected or treated mammalian cells, implanted cells or cells obtained from the primary site in the host animal. DD-PCR products may be analyzed by any method which reliably compares the products of two polymerase chain reactions. Typical analytical methods used for this purpose include polyacrylamide gel electrophoresis, capillary electrophoresis and high pressure liquid chromatography (HPLC). Product produced from DD-PCR may be analyzed in double-stranded or single-stranded forms. When the products of the DD-PCR reaction are labeled the sizes and distribution of the products may be monitored and analyzed by following the labels using a radiation monitor or by autoradiography. For example, DD-PCR performed in the presence of radioactive primers or nucleotide triphosphates, can be analyzed by gel electrophoresis, by capillary electrophoresis, or by HPLC. Products are easily monitored by the presence of radioactivity.

Another method for analyzing and isolating metastatic sequences is to sequence the amplified nucleic acid sequences. Sequencing may be performed using standard methods well known to those of ordinary skill in the art. The resulting sequence may be compared to a sequence database created or well-known, such as Genbank, for identification or for locating homologs. The sequencing information may be used to calculate the physical characteristics of the nucleic acids such as melting temperature and secondary structure. The primary sequence and the physical characteristic may be used to synthesize optimal nucleic acid probes for the detection or staging of metastasis or conditions that are predictive of the presence or absence of the metastatic condition.

Another embodiment of the invention is directed to a method for identifying a metastatic sequence. A mammalian cell is pretreated with a metastatic agent to form a population of cells predisposed to metastasize. The treated cells are introduced into a host mammal at a primary site. The host animal is maintained for a period of time sufficient to develop a metastasis at a secondary site. Expressed sequences of cells at the primary site and cells at the secondary site are treated with a genotoxic agent or subjected to genotoxic conditions. Expressed sequences of the treated cells are amplified by differential display polymerase chain reaction and compared with untreated cells from any previous step to identify the metastasis sequence.

The metastatic agent may be a chemical compound, a nucleic acid or a protein that alters the metastatic potential of a cell or relates to or is associated with the metastatic process. Chemical compounds include retinoids such as 4-hydroxyphenyl (4HP). Other agents include the proteins TGF-β1, Cyclin D1, p21, p34, mutant p53, lysyl oxidase, caveolin, actin binding protein, ubiquitin activating enzyme E1, nmb or α-actinin 3, or their respective genes. The metastatic agent may be a metastatic stimulant or a metastatic suppressant. Metastatic stimulants may be used to enhance the sensitivity of the metastasis sequence detection method. Conversely metastatic suppressants may be used to decrease the sensitivity of the method enabling the selective identification of potent metastatis sequences or sequences specific to a particular tissue type or metastatic disorder. Treatment may comprise direct contact with the metastatic agent or incubation for a period of time. Metastatic agents enhance the metastatic potential of the implanted cells and increase the sensitivity and the speed of the overall method.

The cells at the primary site and the metastatic cells at the secondary site may be treated with a genotoxic agent *in vivo* or *in vitro*. *In vivo* treatment may comprise injecting genotoxic agents directly into the host mammal or specifically applying the agent with, for example, topical formulations. The cells at the primary site and the secondary site may also be isolated from the host animal and treated with the genotoxic agent in culture. Genotoxic agents are chemical compounds, nucleic acids or proteings that alter gene expression by effecting the nucleic acid genome directly by, for example, chemical modification, or indirectly by, for example, altering components associated with gene expression. Such agents include, for example, benzanthracene (BA), dimethyl benzanthracene (DMBA) and 5-azacytidine, and may include metastatic agents as well. In addition to or in place of genetoxic agents, the cells may be treated to hypoxic conditions or radiation to alter gene expression. Metastatic sequences identified in these methods may be specific for particular genotoxic agents or conditions.

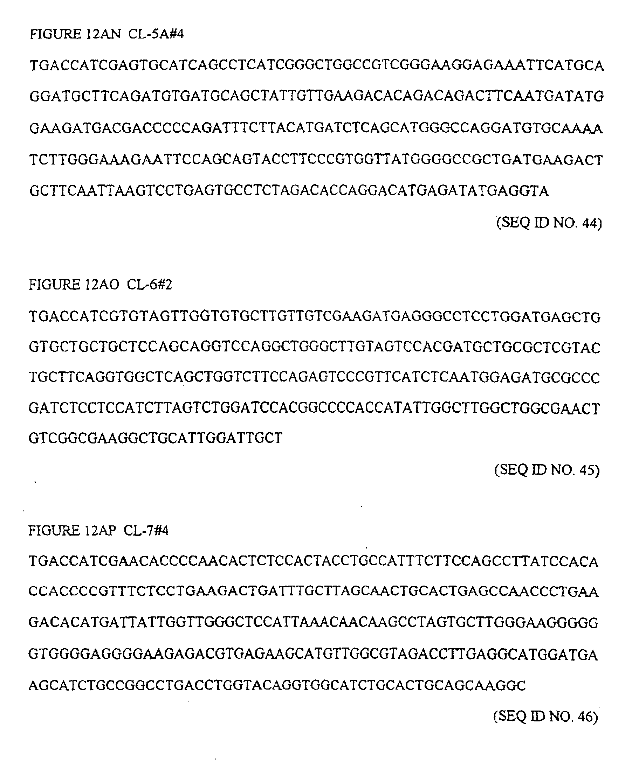

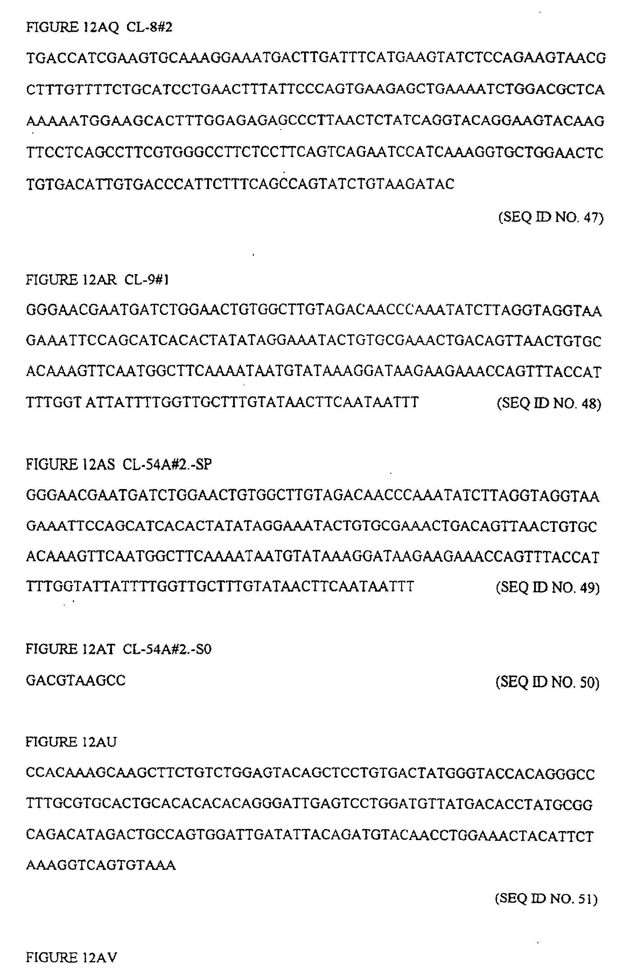

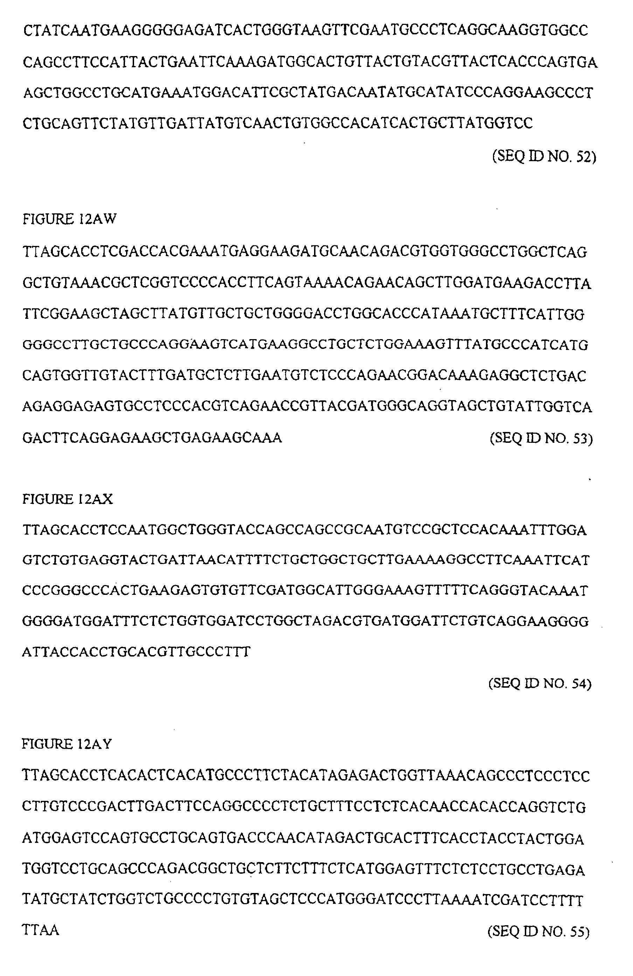

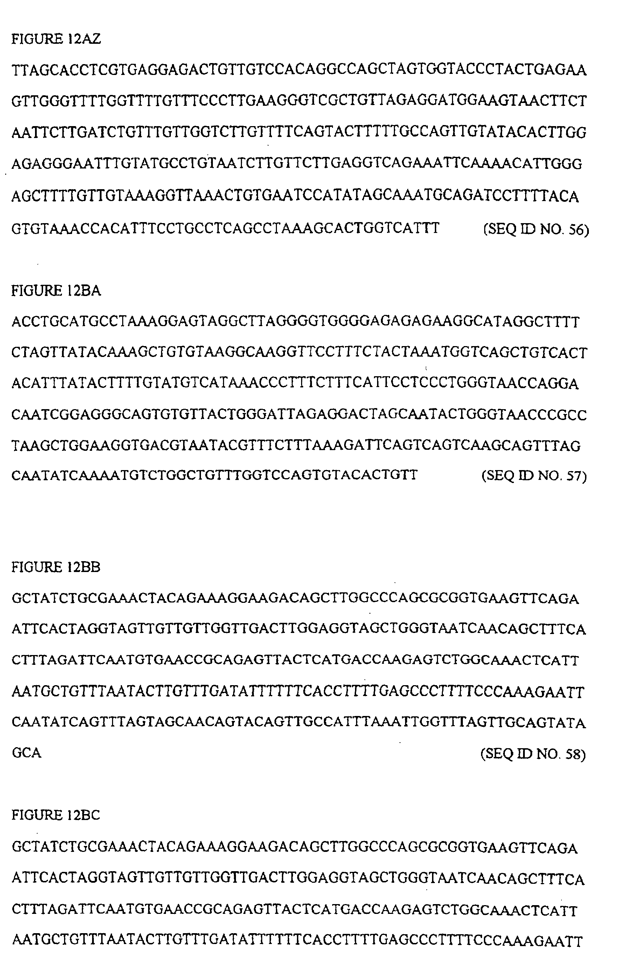

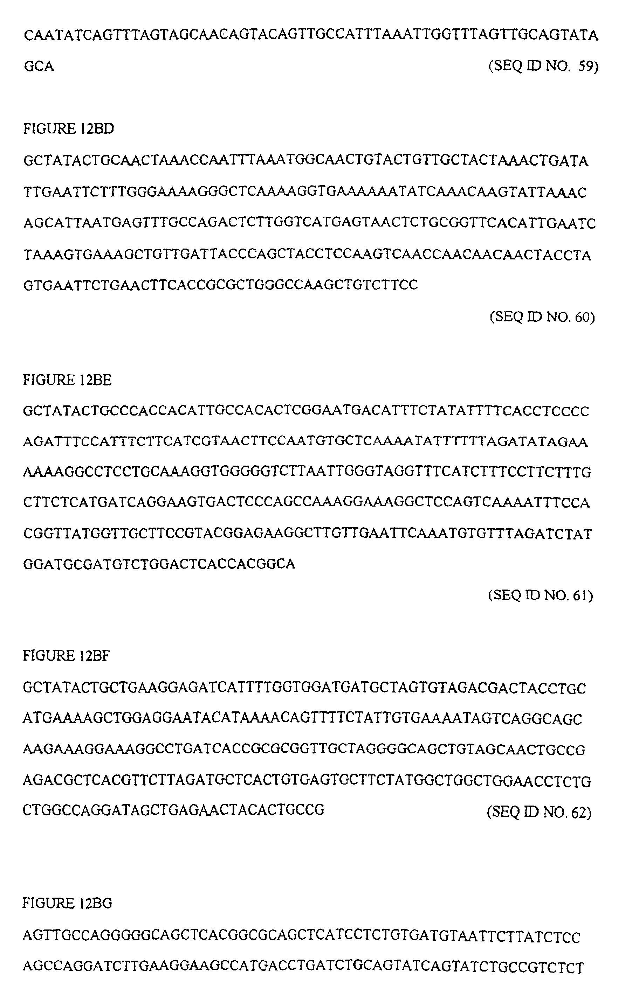

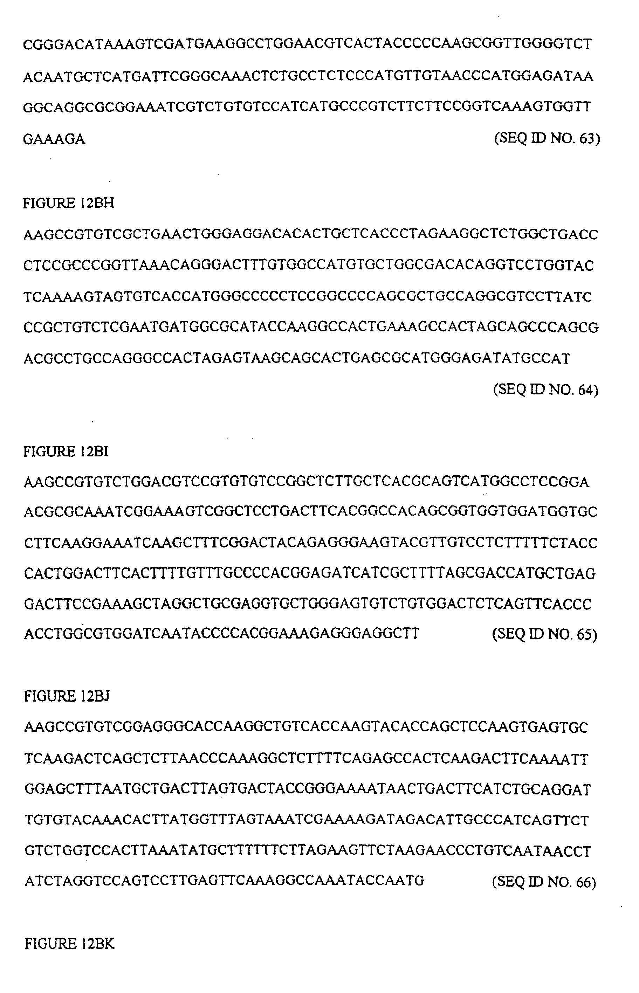

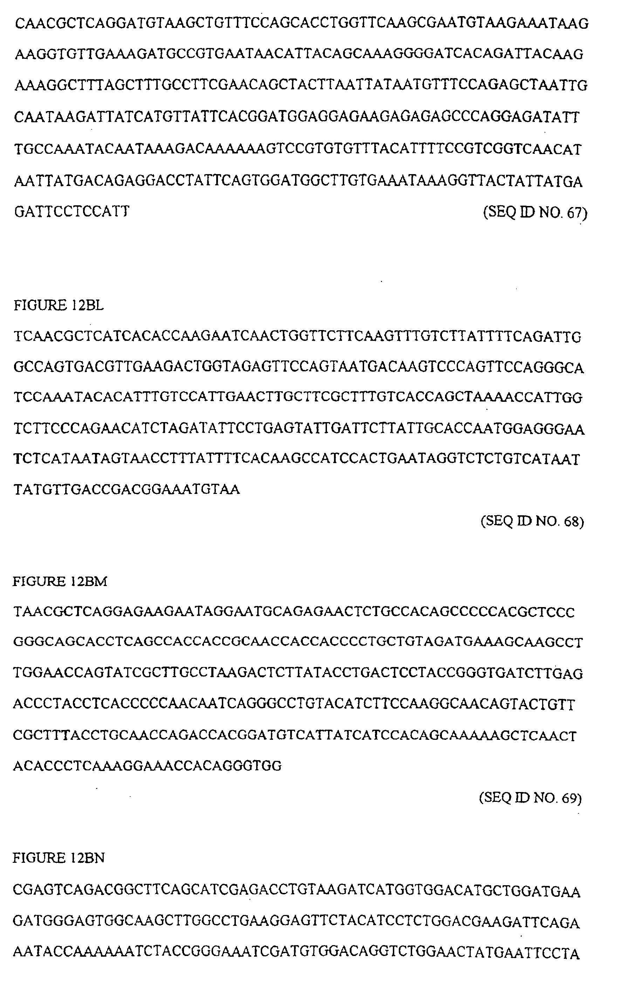

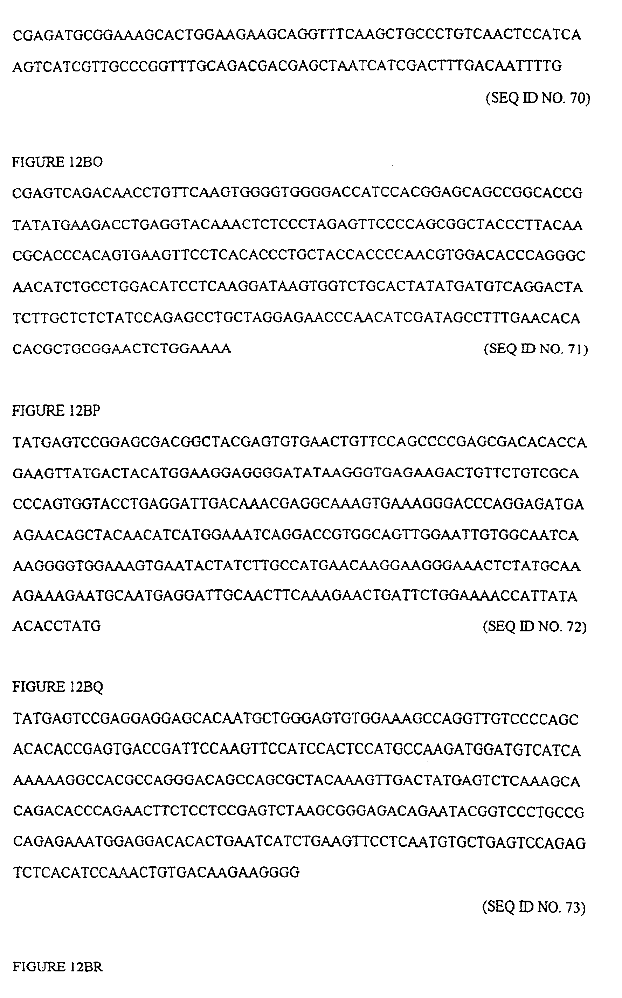

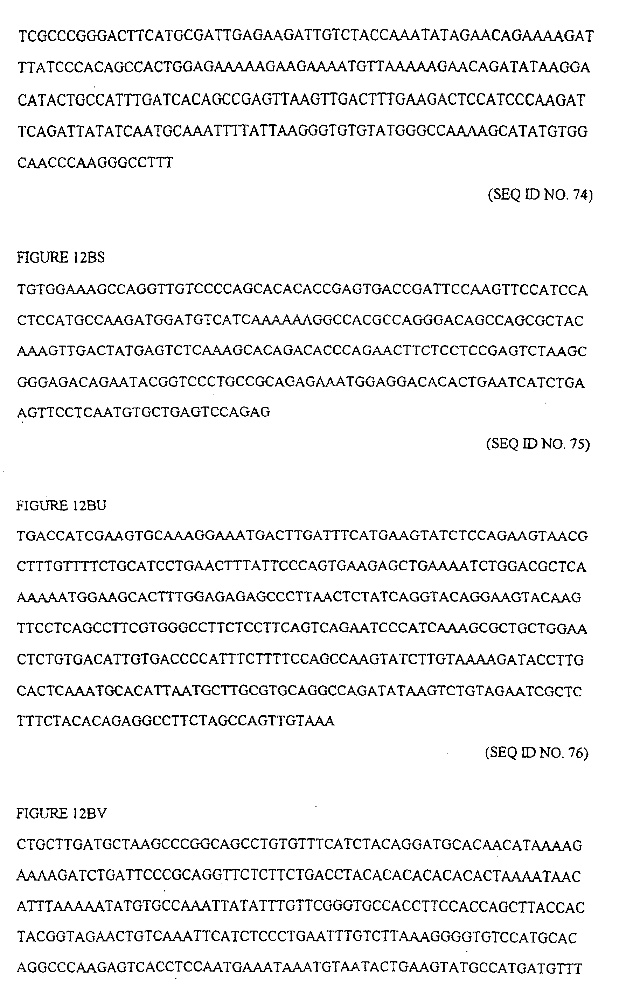

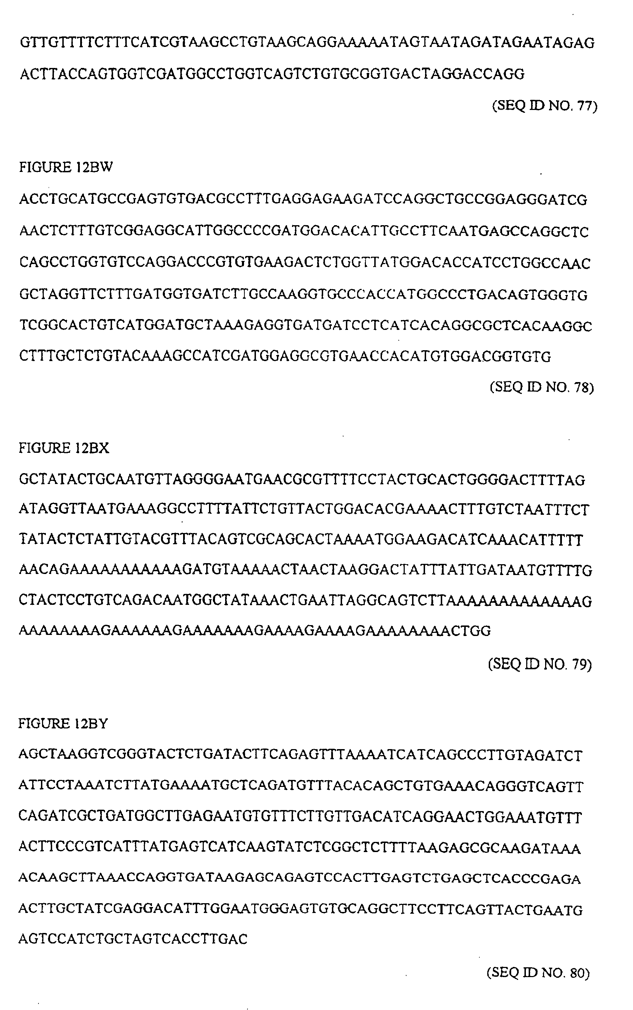

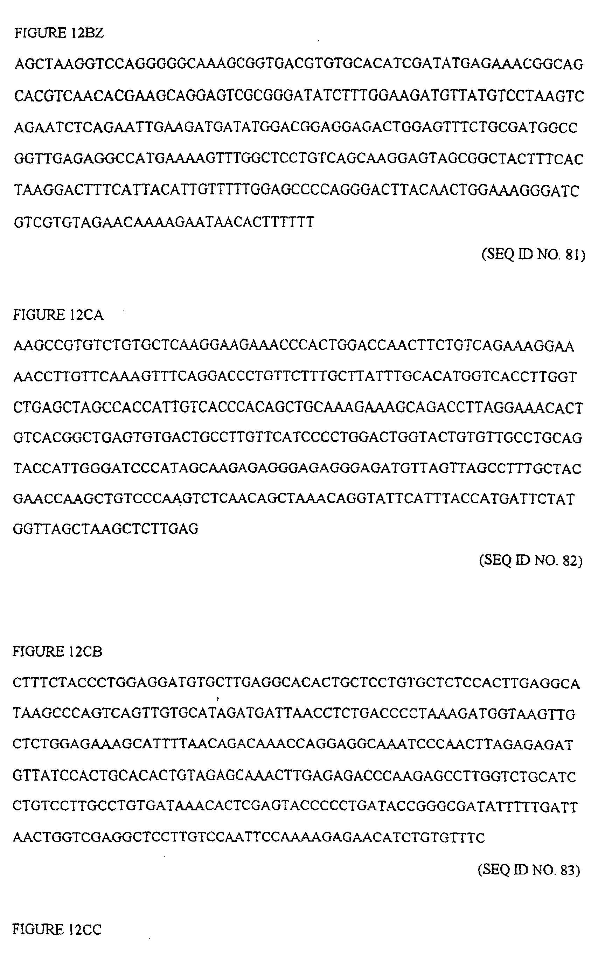

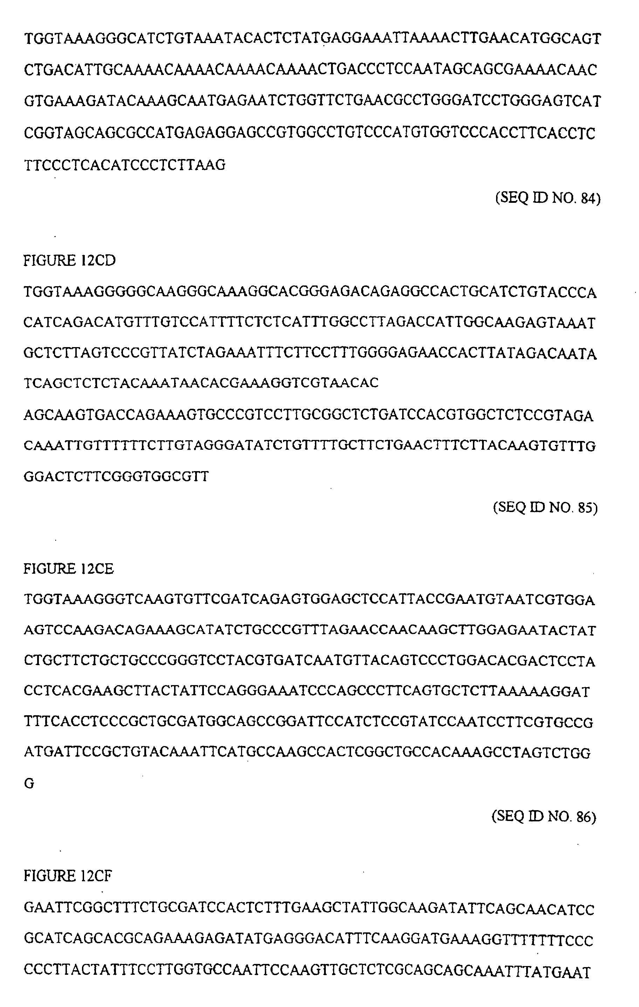

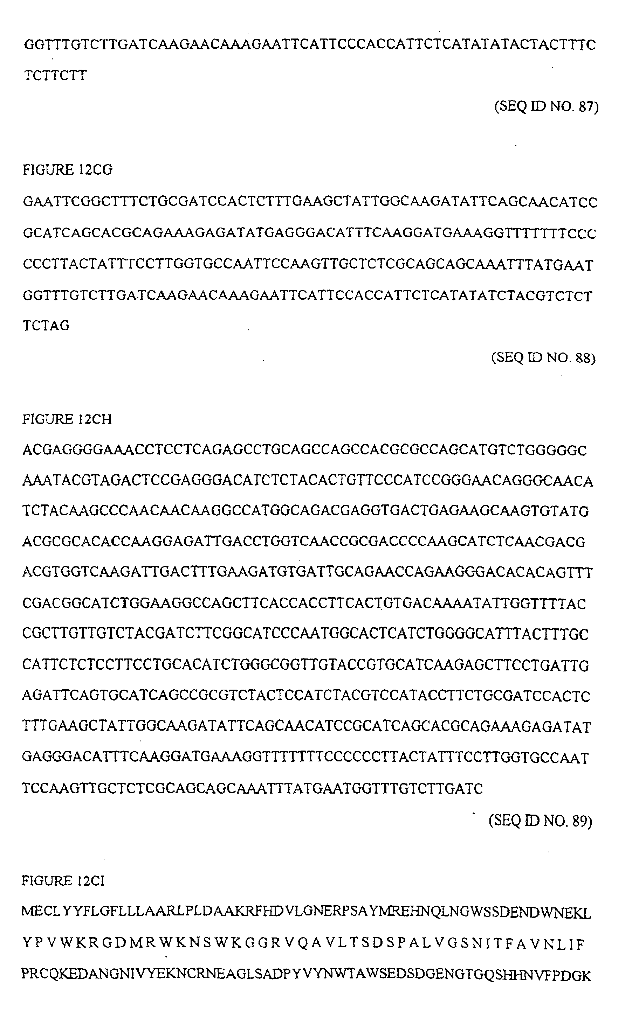

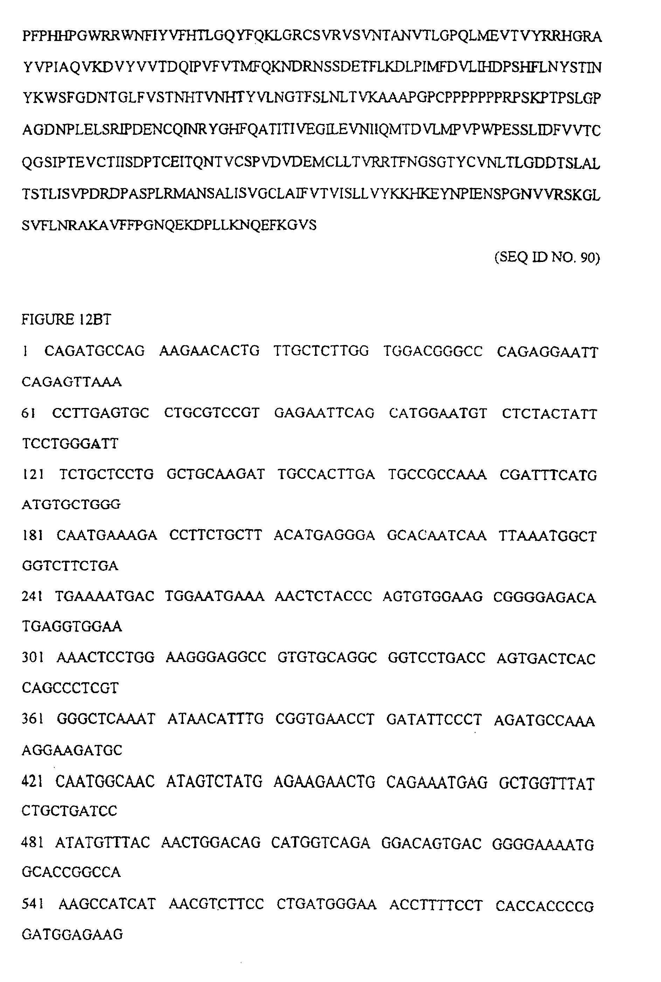

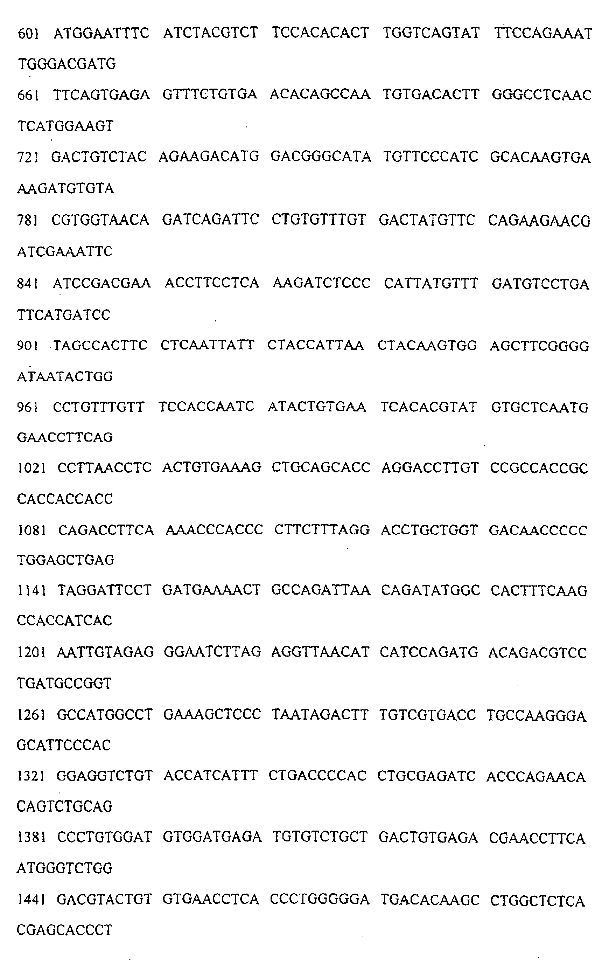

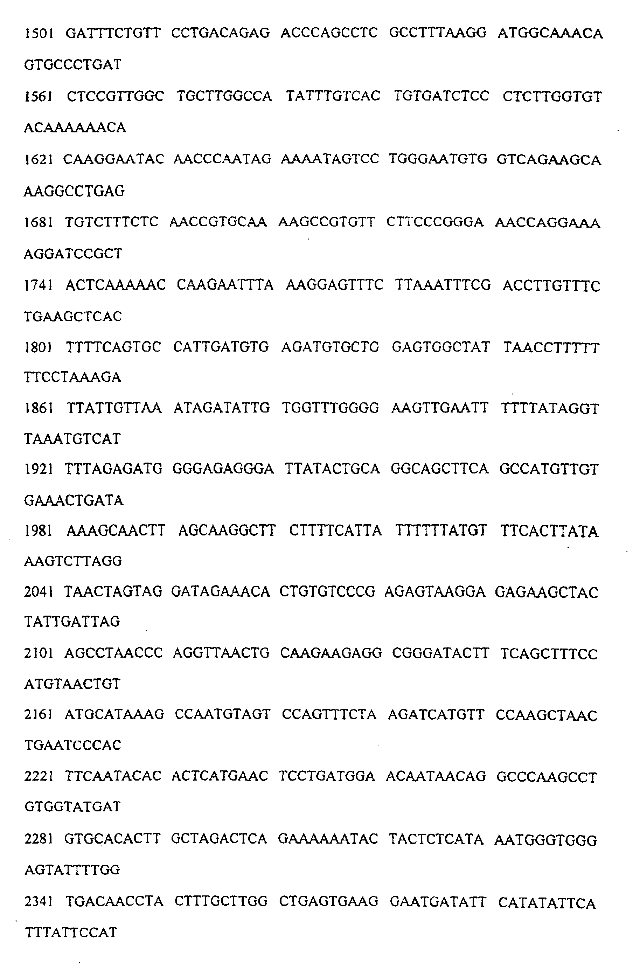

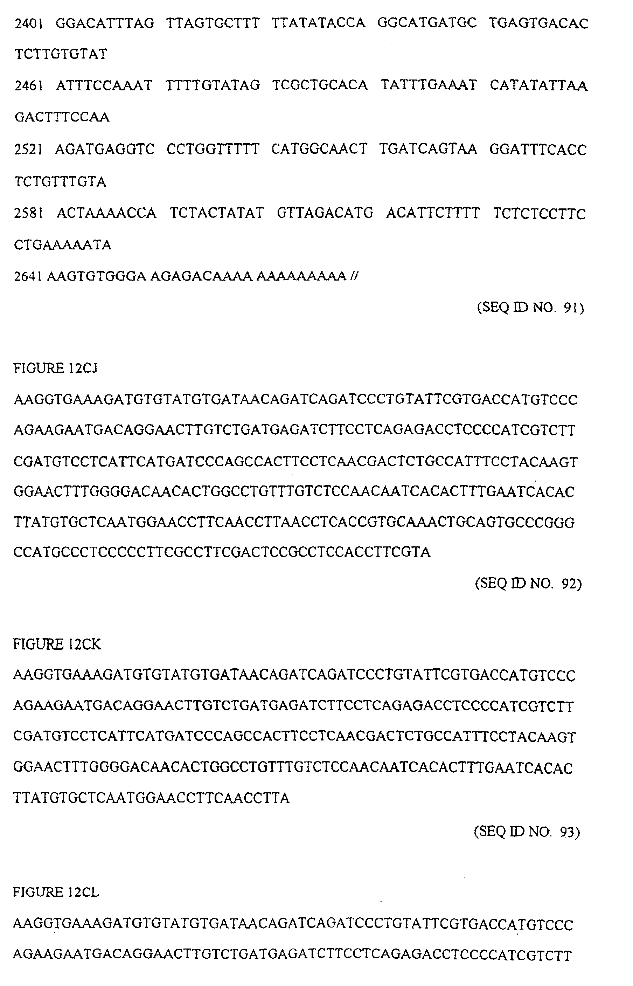

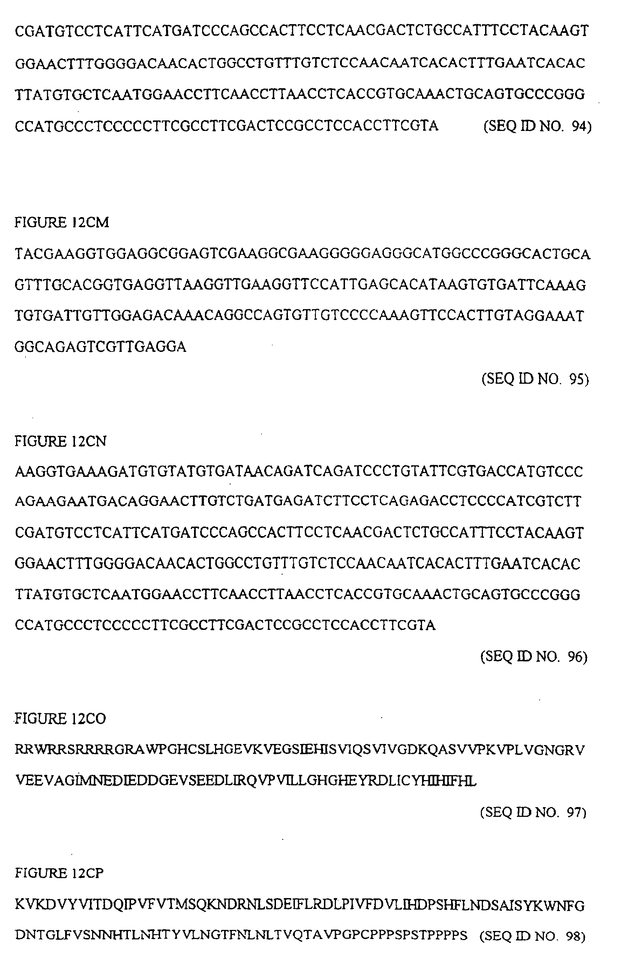

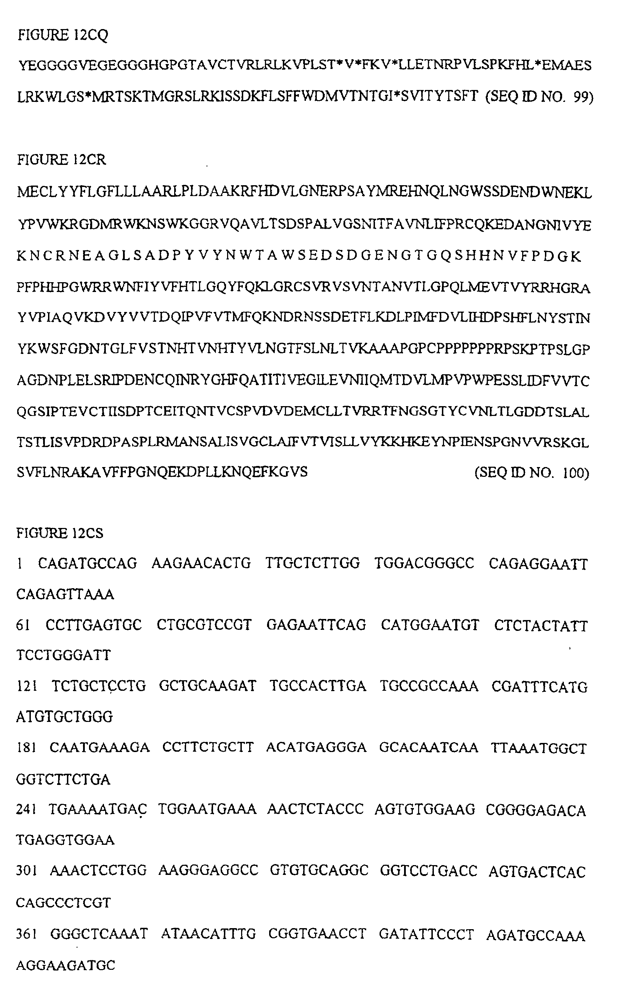

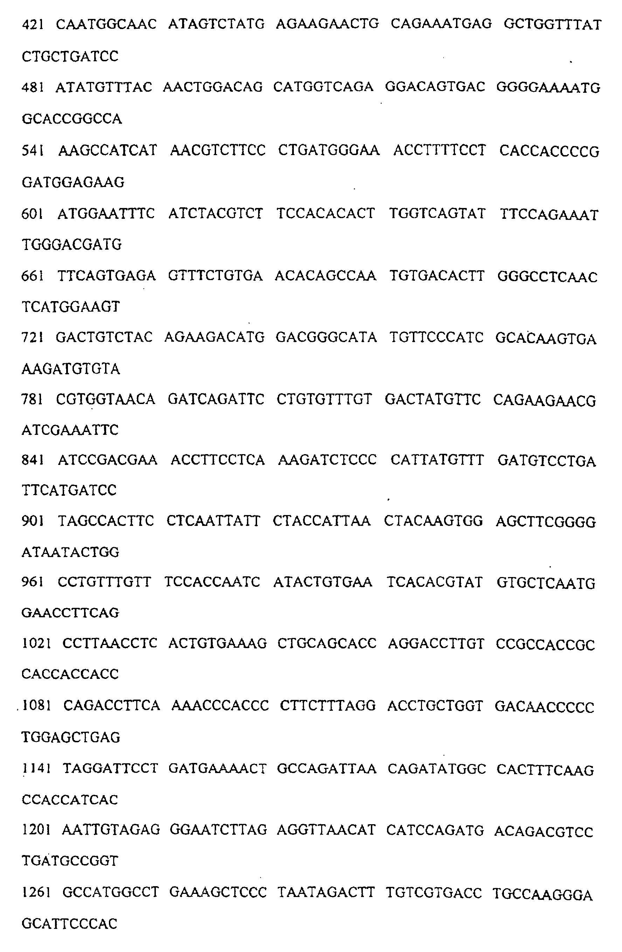

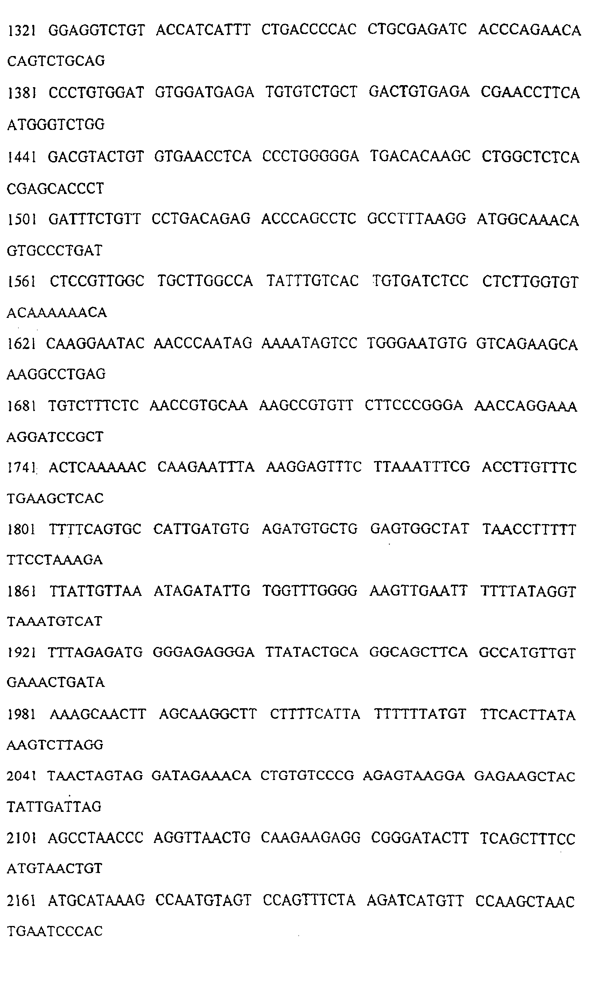

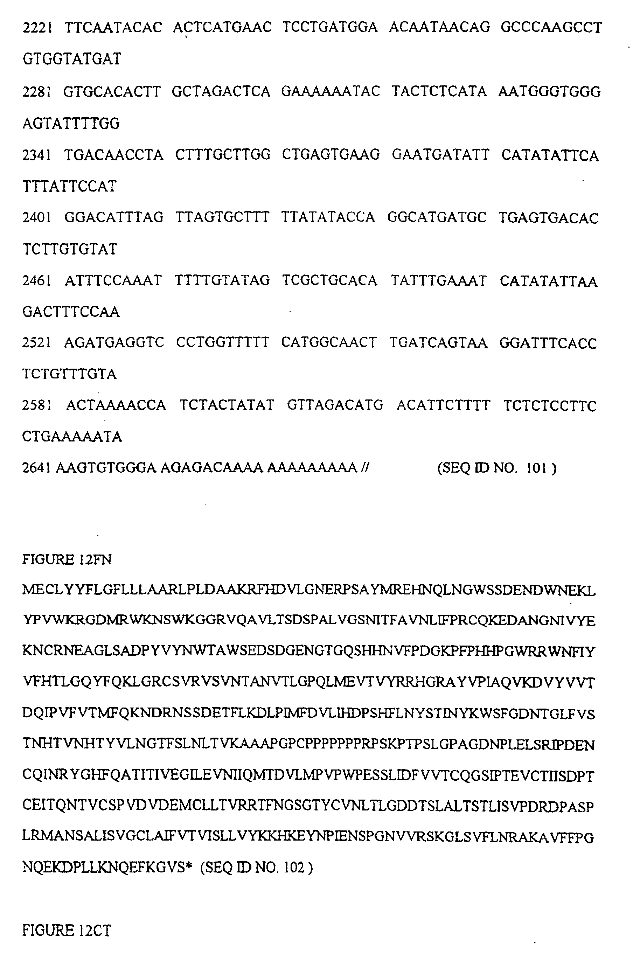

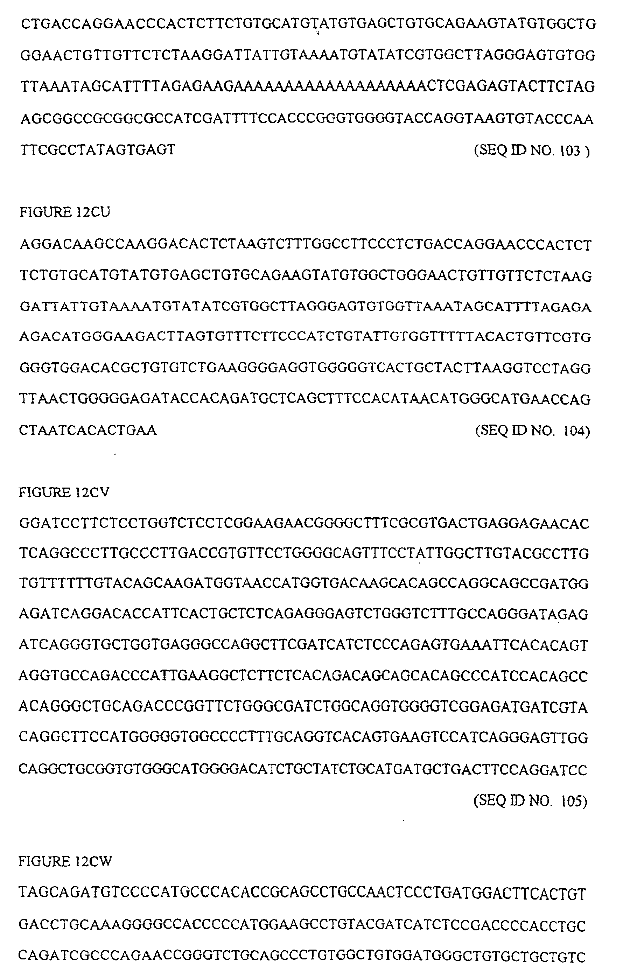

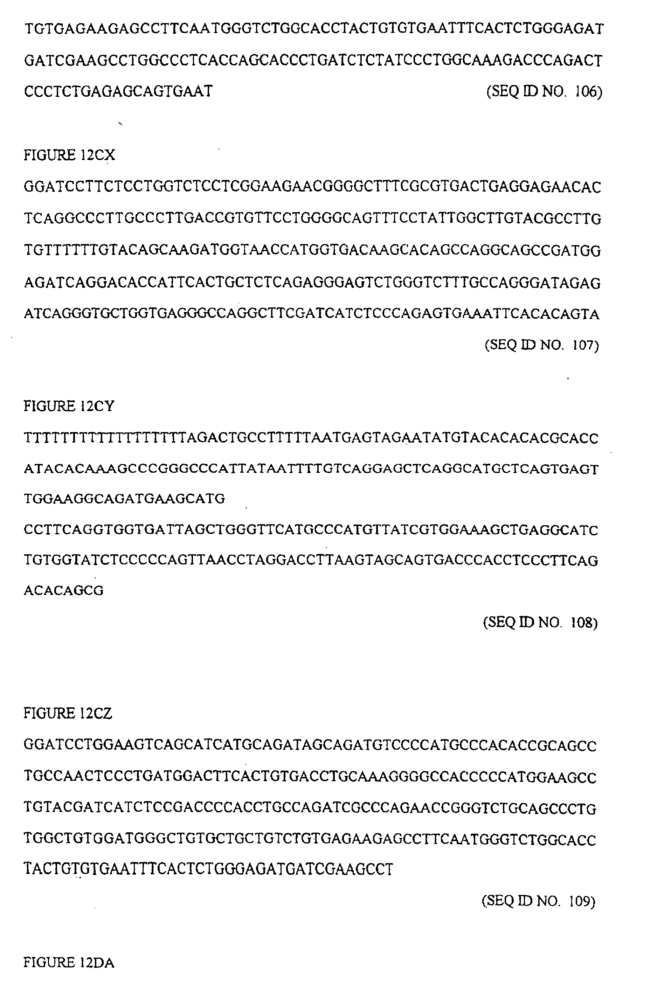

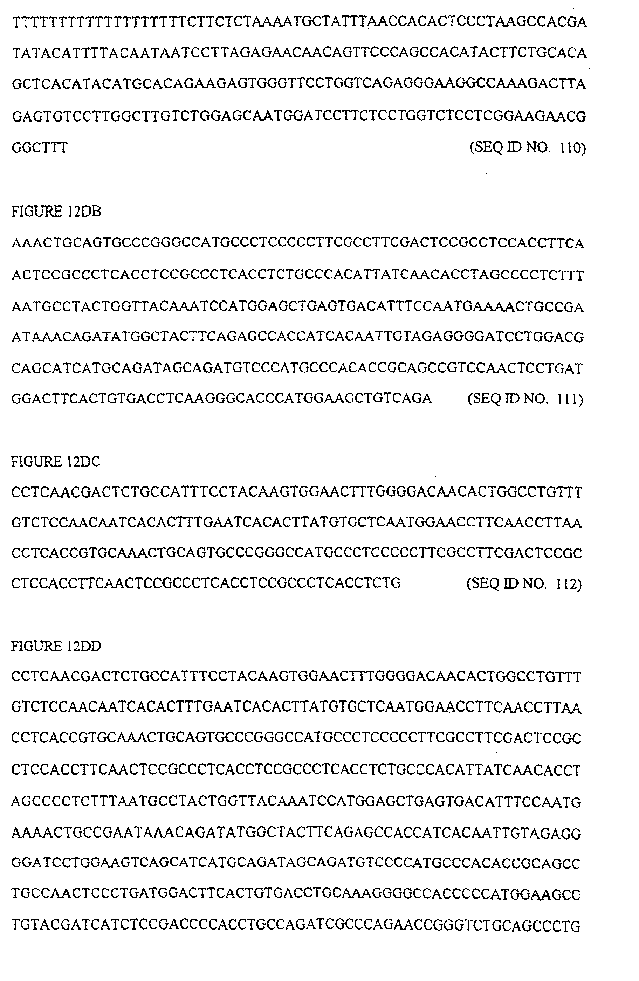

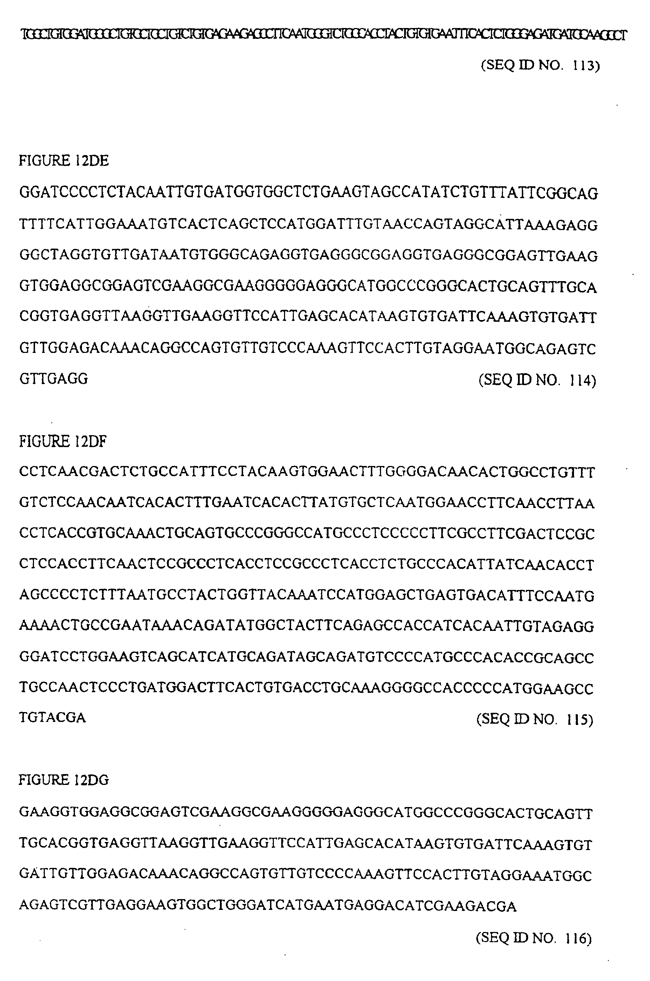

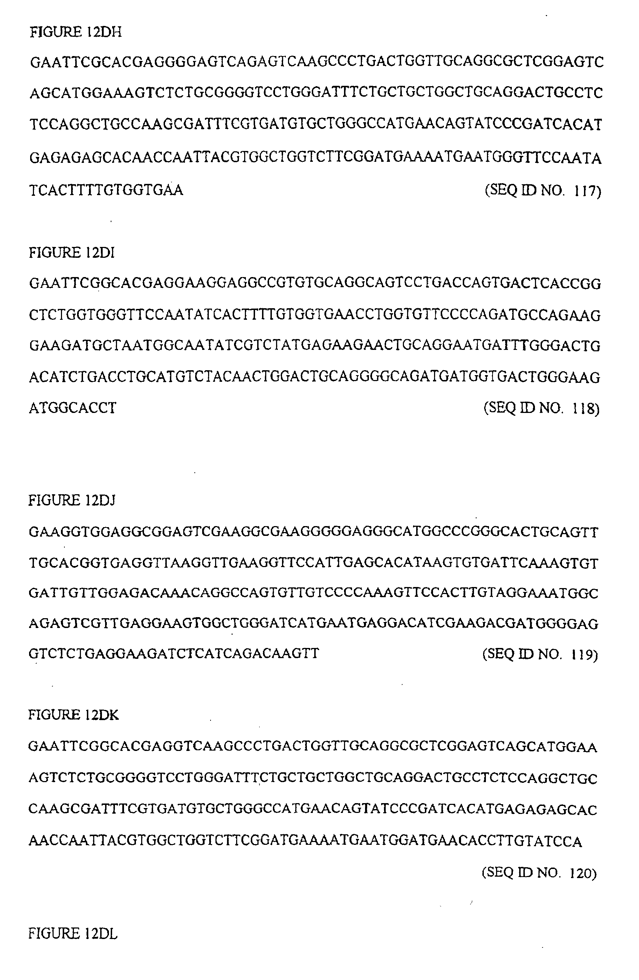

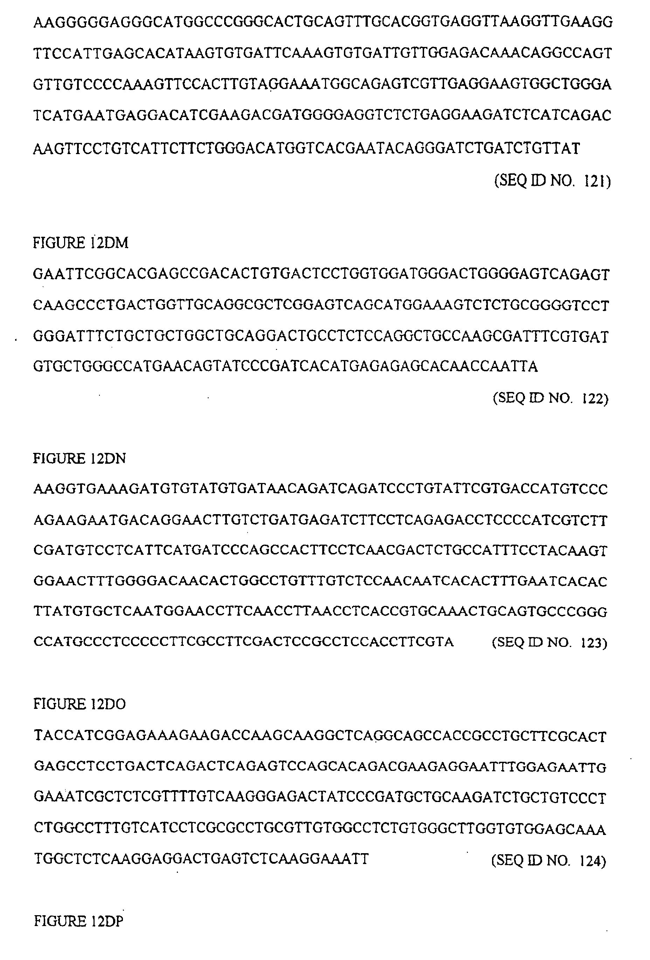

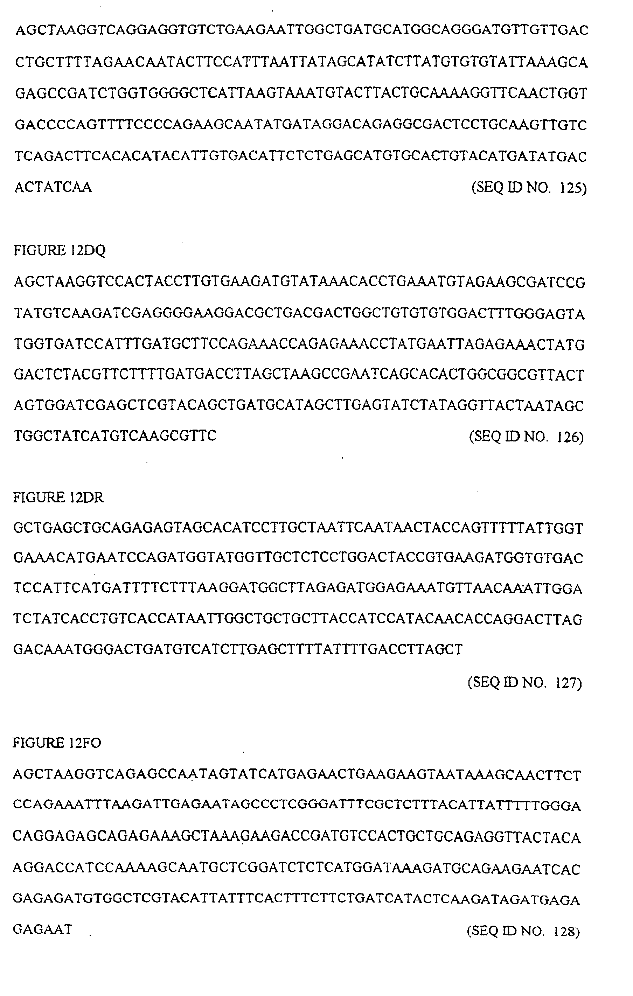

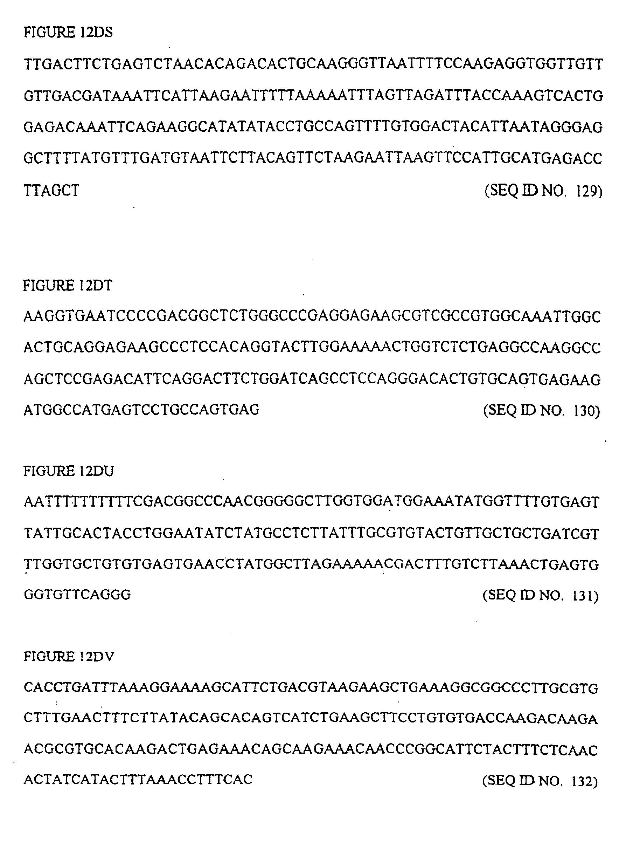

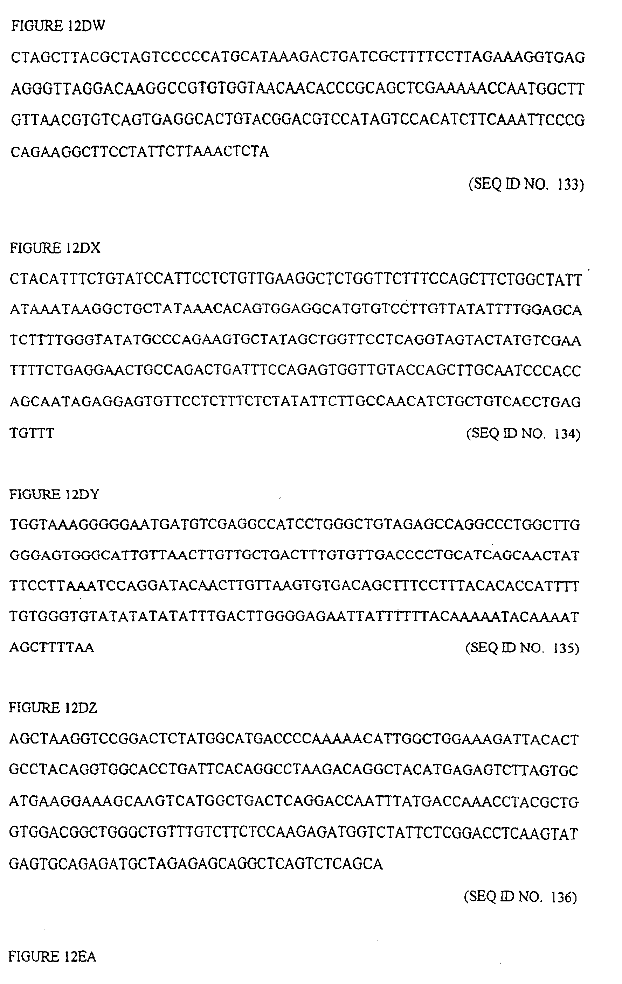

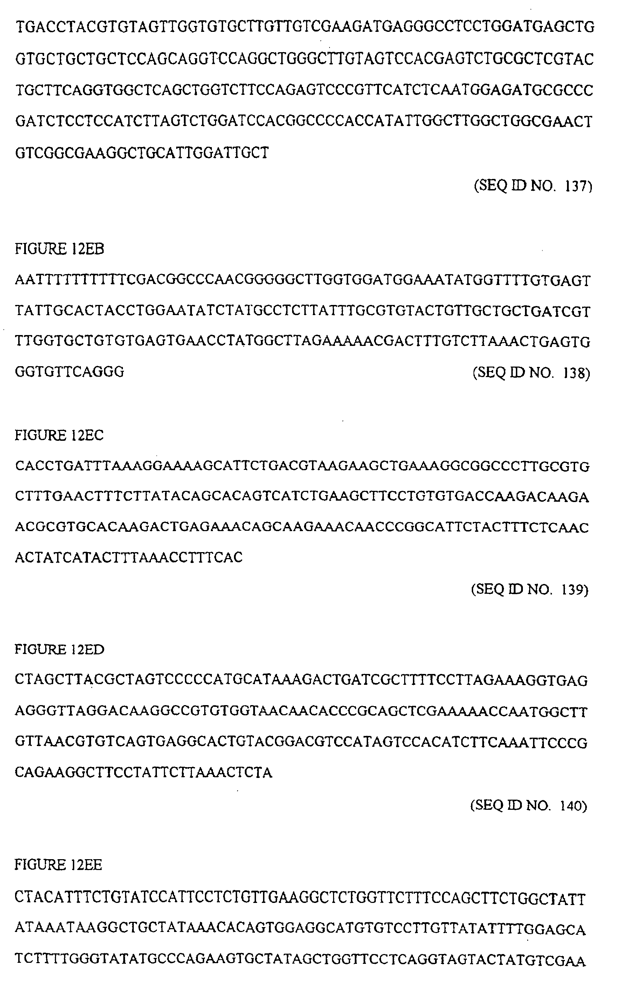

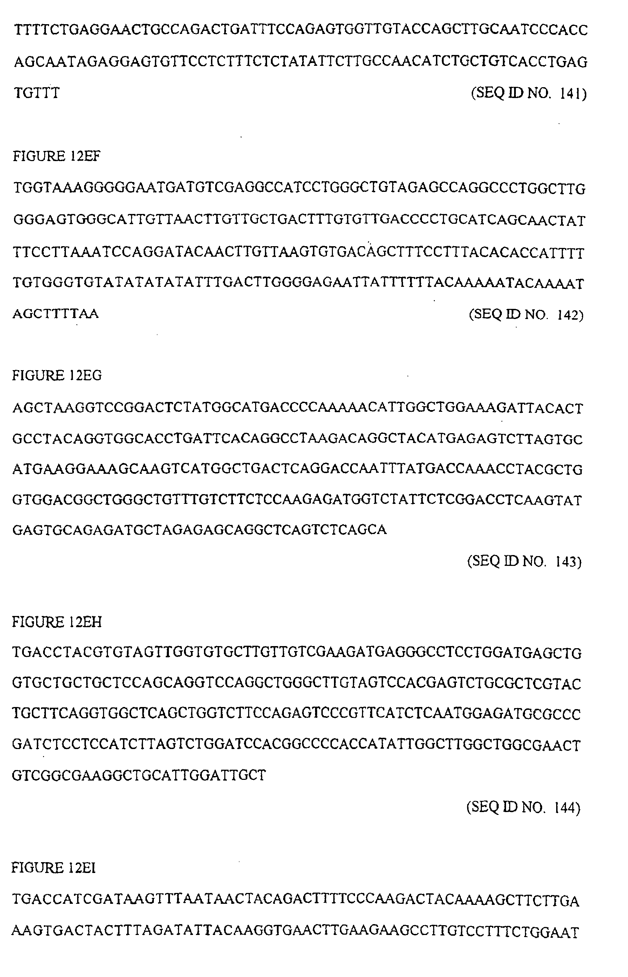

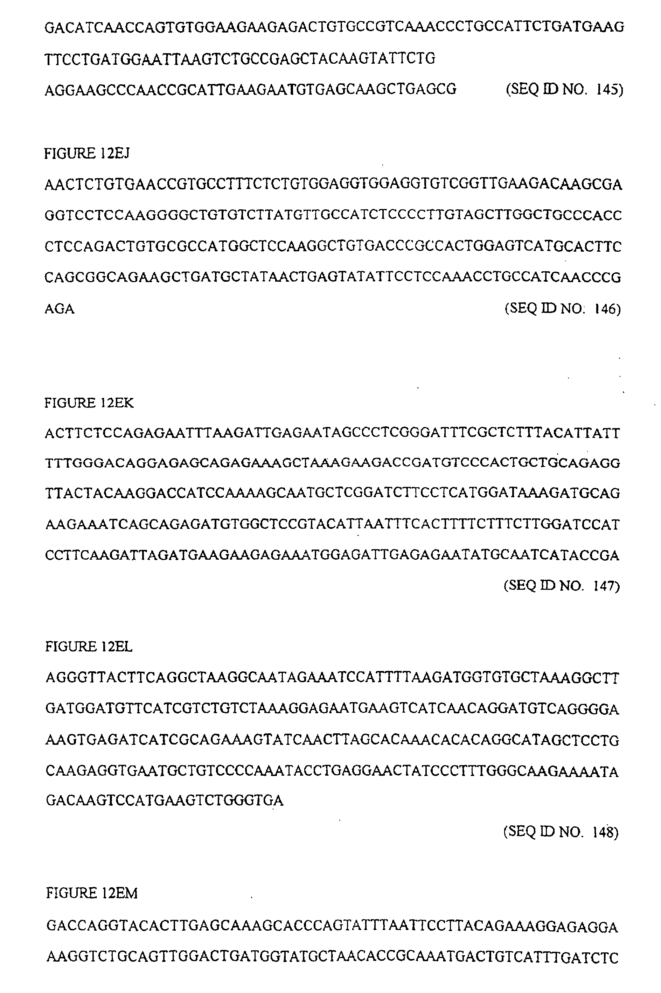

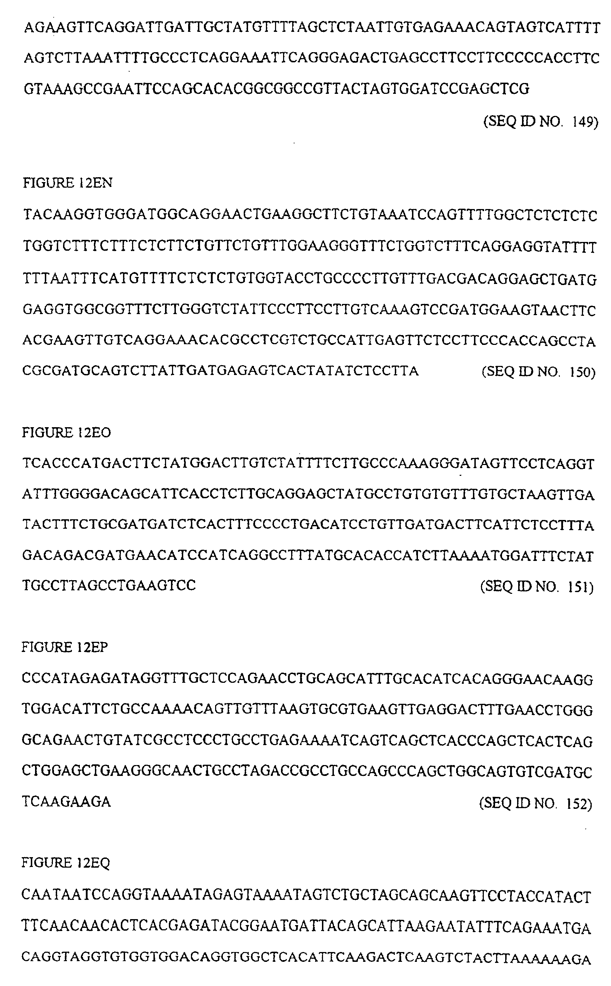

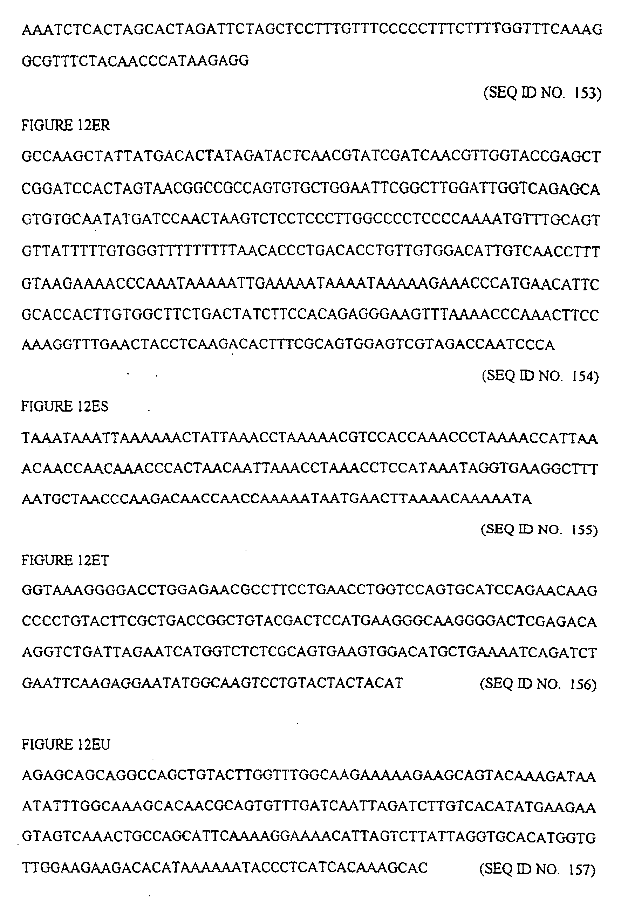

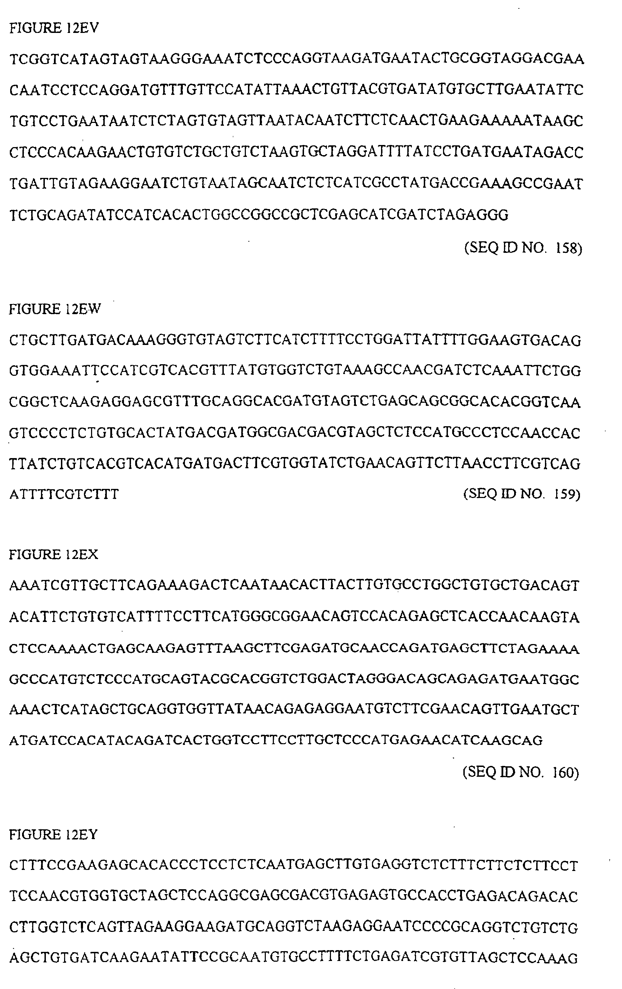

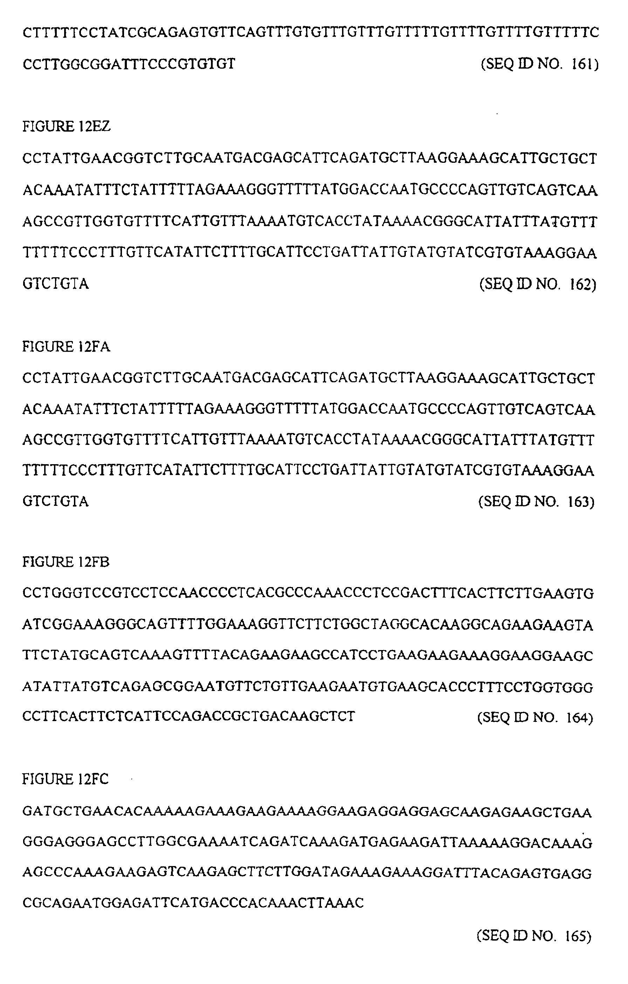

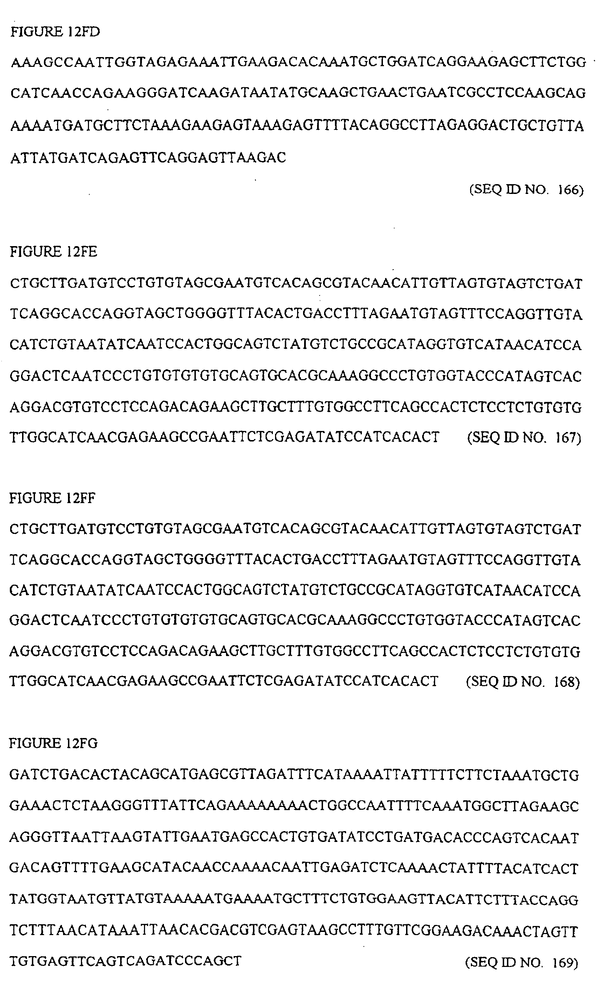

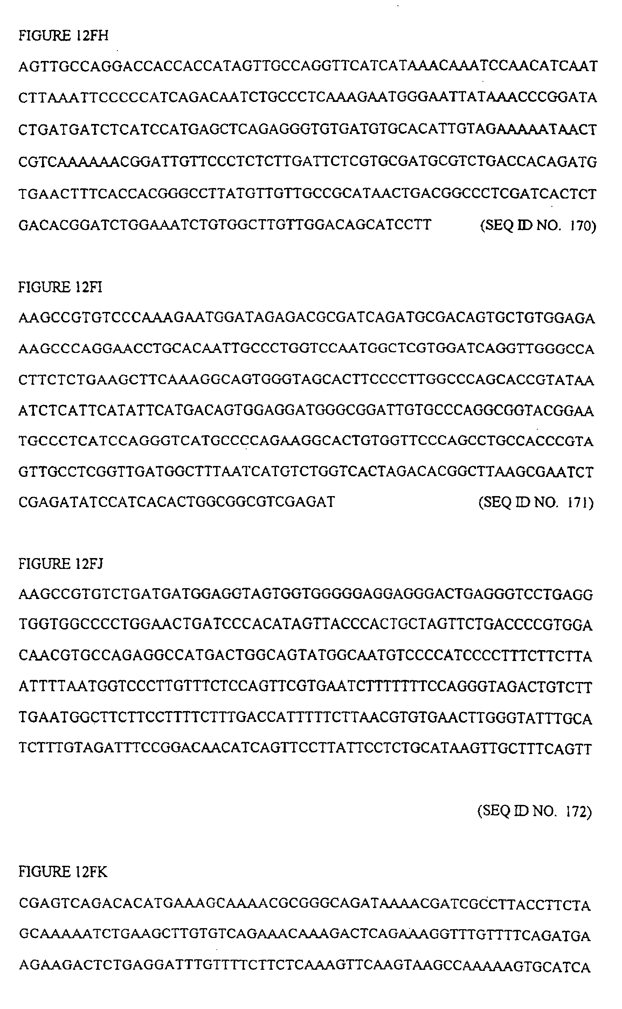

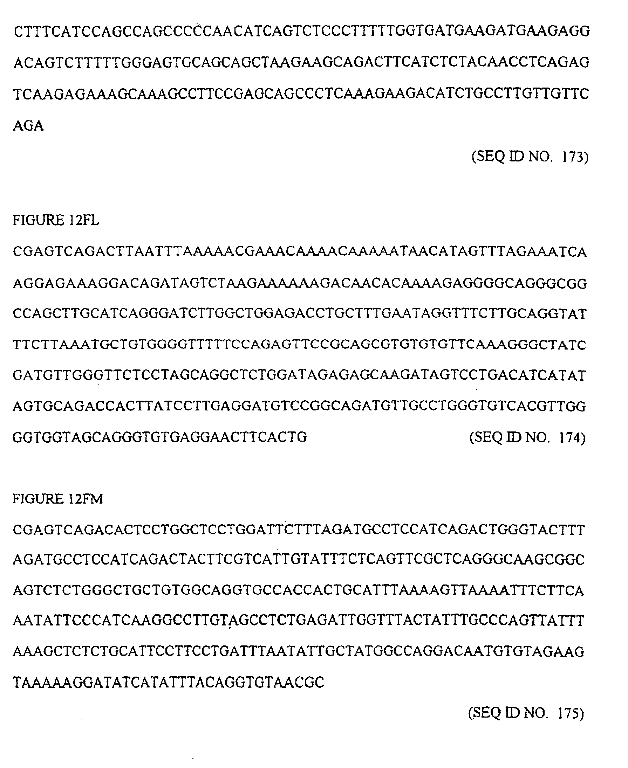

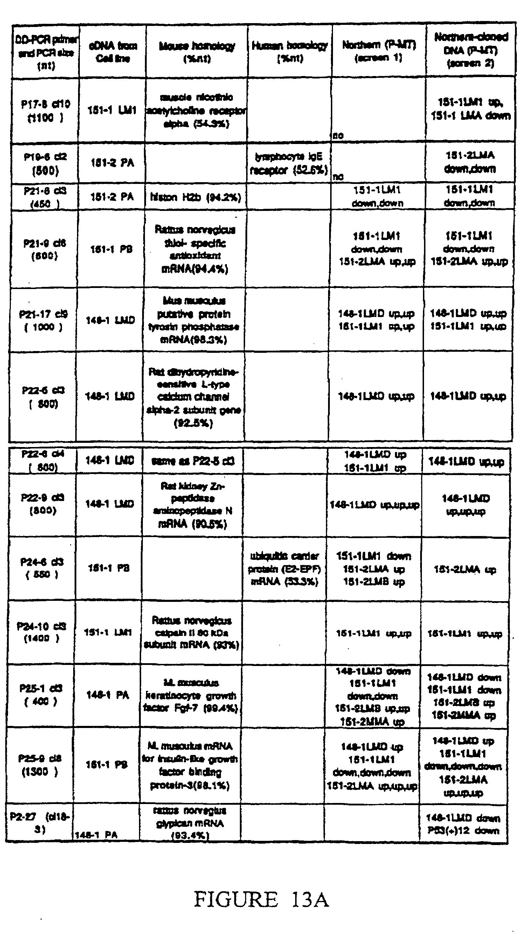

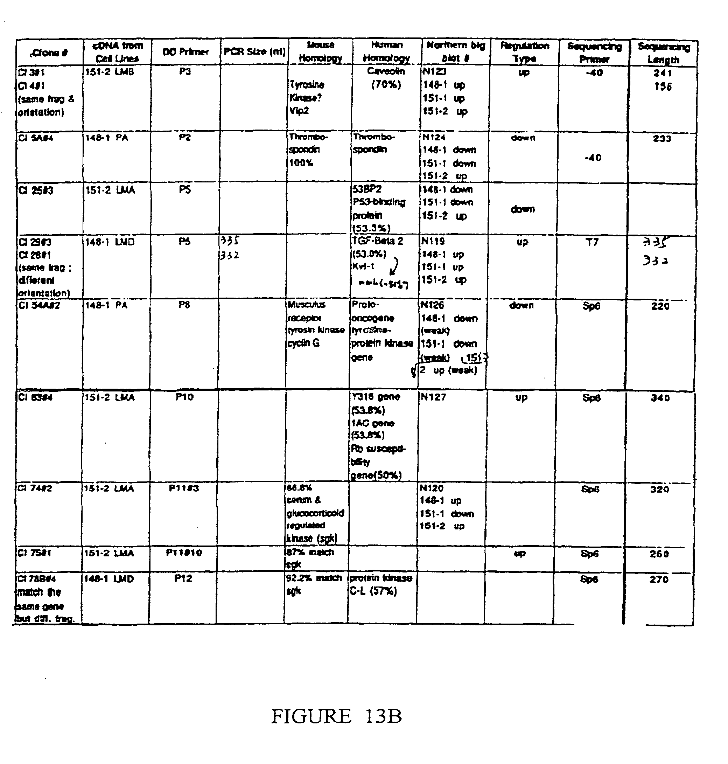

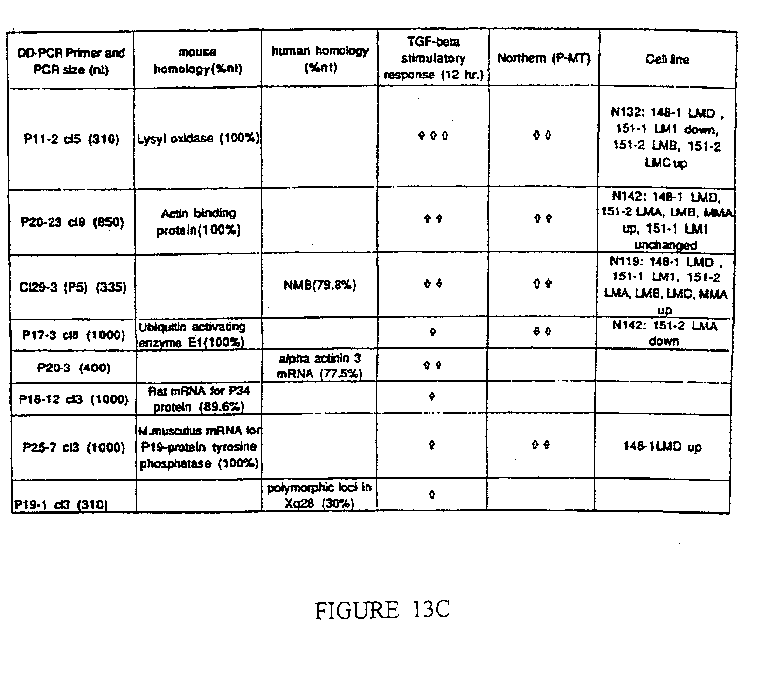

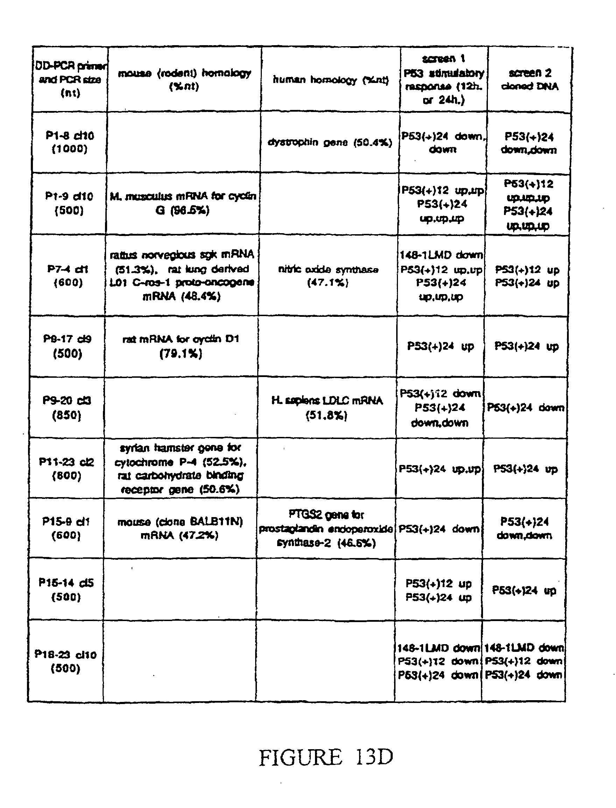

A host animal with an altered genotypic or phenotypic predisposition for metastases may be used. A host animal may be screened for endogenous expression of metastases gene. Examples of metastatic sequences which may be screened for include sequences isolated by the method of the invention, such as, for example, the sequences listed in Figure 12 and Figure 13. Particularly useful metastatic sequences include *TGF-β*. A host animal with reduced levels of a metastatic gene product may be used to isolate novel metastatic genes. Host animals may be screened for reduced levels of metastatic gene expression. In addition, transgenic technology may be use to ablate a metastatic gene in the germline of a host animal.

Analysis is useful in identifying cells, and consequently sequences specific to these cells, which are particularly susceptible or resistant to metastatic transformation. For example, a cell highly predisposed to metastasis may be especially sensitive for detecting metastatic genes. Conversely, a cell showing high resistance to metastasis can be used to isolate especially potent metastatic sequences. One method to analyze susceptibility to metastasis is to determine the cellular response to growth factors or growth inhibitors. Briefly, a control population and a test population of cells are exposed to a growth factor or a growth inhibitor and the cellular response (e.g. proliferation, metabolism) recorded. Cells showing abnormal responses to the growth factor or growth inhibitor may be used as the starting material for metastatic gene isolation. Cellular response include changes in the rate of cellular division (e.g. thymidine uptake), changes in the expression of RNA or proteins, changes in cellular localization or modification patterns of RNA or proteins, and changes in the rate of uptake, release or metabolism of nutrients.

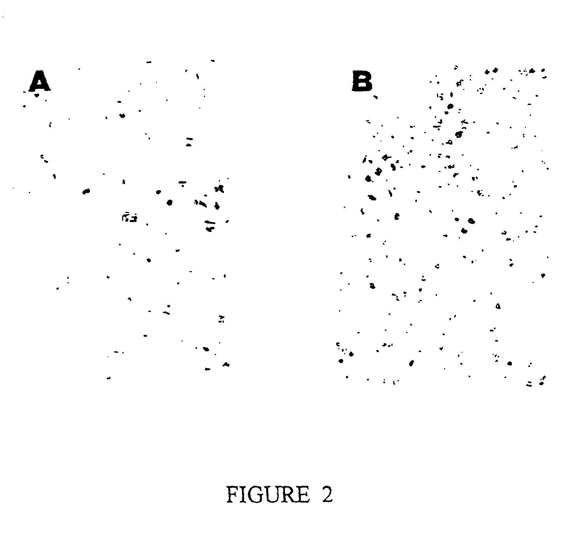

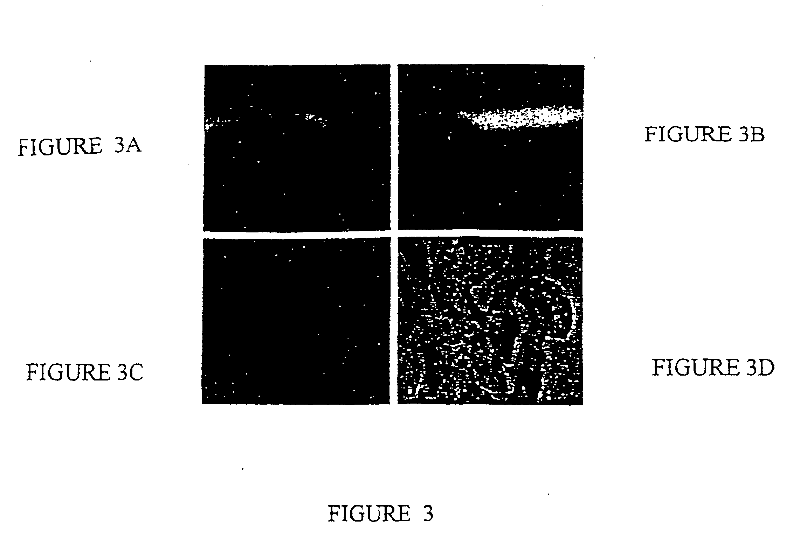

Especially potent or weak metastatic genes may be detected by treating and analyzing the metastatic potential of different cells and selecting a suitable cell type as the starting material. For example, cells may be treated with *myc, ras, mutant p53* or combinations thereof and analyzed for *cyclin D1* expression which is shown to correlates with metastasis. Figure 2 shows the *in situ* analysis of *cyclin D1* in primary MPR tumors (Figure 2A) and in metastatic deposits from the lung of the same animal (Figure 2B). The gene expression pattern of *cyclin D1* in MPR correlates with that of human prostate tumors (Figure 3) analyzed with stains specific for *cyclin D1* expression. Normal human tissue shows no *cyclin D1* expression or staining (Figure 3A). Moderately differentiated prostate cancers with dispersed (Figure 3B) or focal positively staining (Figure 3C) show moderate staining. Advanced poorly differentiated prostate cancer show strong nuclear as well as cytoplasmic staining (Figure 3D) implying strong expression of *cyclin D1*. After treatment with *myc, ras* or *mutant p53, cyclin D1* expression shows correlation with the metastatic potential of the cell. Thus, *cyclin D1* expressing cells are a source of cells with high metastatic potential. Conversely, cells with low *cyclin D1* expression are a source of potentially metastatically resistant cells.

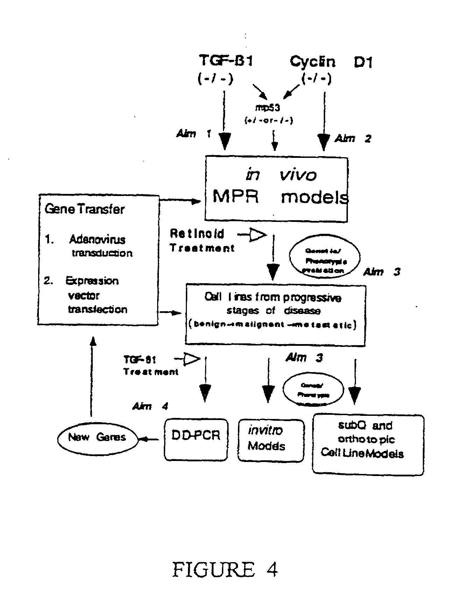

This method may be adjusted for the isolation of metastatic sequences expressed along a particular developmental or differentiation pathway by combining the various treatment and analytical techniques. This approach is schematically represented in Figure 4. For example, a mammalian cell may be genetically ablated for *TGF-β1, Cyclin D1, mutant p53, lysyl oxidase, caveolin, actin binding protein, ubiquitin activating enzyme* *E1, nmb,* α *actinin 3,* or *p34*. The genetically altered cell is used in *a in vivo* mouse prostate reconstitution (MPR) model. Metastatic and nonmetastatic cells isolated from the MPR may be analyzed directly or after induction with an agent such as the *TGF-β* gene or its product. Analysis involves the use of differential display polymerase chain reaction to identify differentially expressed bands. Sequences identified may be used for subsequent ablation, transformation or differential analysis.

Genetic ablation (gene knockout) may be performed after a cell is selected or by selecting a cell comprising a genotype with the proper genetic ablation. Cells already comprising gene ablation may be acquired from a cell depository, from other laboratories or from a transgenic animal. As transgenic animals comprise genetically ablated genes in every cell, any tissue from a transgenic animal may be used as the starting material.

The effects of oncogenes are at least additive and often synergistic. Thus, dominant oncogenes may be transfected together or multiple recessive oncogenes ablated together for a stronger effect. Furthermore, both methods may be combined and dominant oncogene transfection may be accompanied by recessive oncogene ablation.

The function of the metastatic sequence may be determined by the differential expression pattern. For example, a dominate metastatic gene will be present in a metastatic cell while a recessive metastatic gene is present in a non-metastatic cell. Metastatic sequences may be detected as bands which are present in the DD-PCR of metastases isolated in secondary sites and absent from DD-PCR products of primary cells. These sequences may be dominant metastatic genes whose expression is directly responsible for metastases, or they may be metastasis associated genes whose expression correlates with metastasis. Either are useful for therapy and diagnosis. Conversely, DD-PCR bands which are present in primary site tumors, but absent in secondary metastatic sites, may be dominant metastasis suppression genes. Dominant metastasis suppression genes comprise genes whose expression suppresses metastasis while nonmetastatic genes comprise genes whose expression correlates with non-metastatic tissue. Genes which are highly correlative with either the metastatic phenotype or the non-metastatic phenotype may be isolated. Isolation can be performed by cutting the appropriate nucleic acid in the band of a polyacrylamide gel or by collecting the appropriate fraction in an HPLC or capillary electrophoresis. The nucleic acid may be cloned into a plasmid vector, and sequenced, or synthetically prepared.

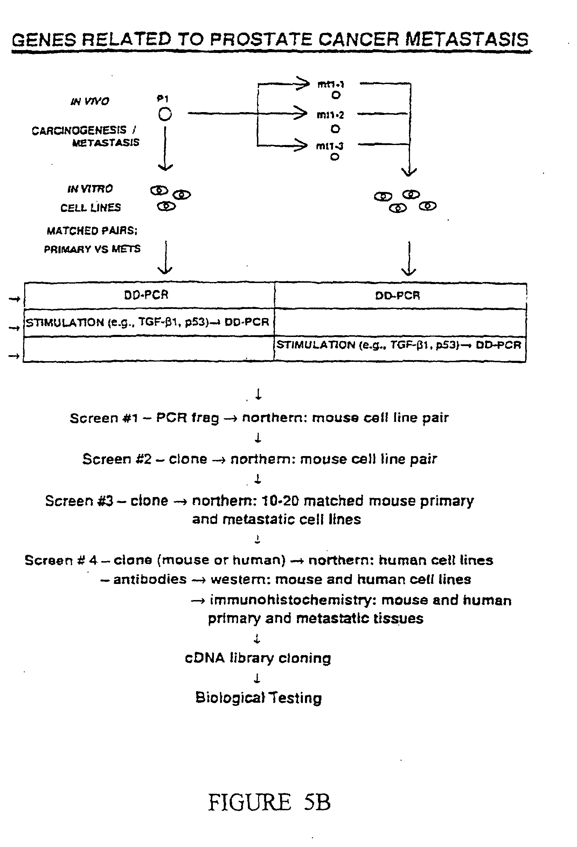

Sequences in a metastatic pathway which are responsive or unresponsive to extracellular signals may be used in therapy and diagnosis of metastatic disorders. Implanted cells or cells from a primary site and cells from a secondary site are treated with extracellular signals. RNA sequences from the treated cells are compared with RNA sequences of the untreated cells (Figure 5B). Treated cells and untreated cells may be derived from a short term or long term *in vitro* culture of primary tumor and malignant tumors. Alternatively, a part of a primary tumor and a part of a malignant tumor may be collected before the animal is treated with an extracellular cytokine or other factor. Long term cultures, or cell lines of primary and malignant cells may also be used as recipients of extracellular growth signal treatment. Suitable signals for each experiment will depend on the cell type. Generally, growth factors, lymphokines, inhibitory factors, migratory factors or hormones may be used. Factors previously isolated and factors associated with or causative or suppressive of metastasis are preferred. Thus, transforming growth factor β1 (TFG-β1) may be used to treat cells before DD-PCR analysis. Proteins encoded by the genes isolated by this method are especially useful for the treatment of cells for the isolation of additional sequences. The identification of one sequence responsive to the extracellular signal pathway allows for identification of additional genes upstream and downstream from that sequence.

Metastatic sequences are sequences associated with the presence or absence of a metastasis or related to the metastatic processor can be used in the therapeutic treatment of metastasis. Metastatic-related sequences include dominant metastatic sequences, recessive metastatic sequences, metastasis associated sequences, dominant oncogenes, recessive oncogenes and cell cycle genes. These genes encode for example, proteins involved in cell cycle, signal processing, DNA replication, growth regulation, inter and intra cellular signaling transcription control and translation control. Isolated sequences are useful in the treatment and for the detection of metastatic and other disorders. Disorders which may be treated comprise diseases involving proteins and sequences which are isolated by interaction with the sequences and proteins isolated by the method of the invention. Both malignant or nonmalignant disorders may be treated. Non malignant disorders include hyperplasia, dysplasia and hypertrophy. Examples of nonmalignant disorders include benign enlargement of the prostate, nodular hyperplasia, and benign prostatic hypertrophy.

Treatment may involve gene replacement, gene targeting, antisense inhibition, gene expression or gene suppression. Gene replacement involves replacing a copy of a defective gene with another copy by homologous recombination. Gene targeting involves the disruption of a cellular copy of a gene by homologous recombination. Antisense inhibition exploits the specificity of hybridization reactions between two complementary nucleic acid chains to suppress gene expression. Cloned genes can be engineered to express RNA from only one or the other DNA strands. The resultant RNA hybridizes to the sense RNA and inhibit gene expression. Gene expression and gene suppression involve the introduction of genes whose expression actively inhibits neoplastic transformation and metastasis.

Nucleic acids which comprise a sequence identified by the methods of the invention. may be DNA, RNA or PNA and may be used as a diagnostic tool in the treatment of neoplastic disorders and malignant tumors. The nucleic acids may comprise additional sequences such as promoters, for expression of a sense or antisense message, recombination sequences for gene targeting, selectable markers for transfections, or replication origins for passage in a prokaryotic or eukaryotic host such as animal cells, bacteria or yeast.

Nucleic acids which comprise sequences identified by the method of the invention such as, for example, the caveolin, ABP280 (actin binding protein 280), the lysyl oxidase gene, and the nmb gene (clone 29), and other sequences listed in Figure 12 and Figure 13 may be used in treatment or diagnosis and in diagnostic kits for screening biological samples for the presence or absence of metastasis or metastatic potential. Treatment may involve using the sequences in gene therapy, including gene ablation, gene expression and antisense suppression. Diagnosis may involve genotypic analysis of samples to determine the existence and expression levels of the expressed sequences.

Another embodiment of the invention is directed to the use of The caveolin gene and protein may be used in the isolation of oncogenes and in the treatment of neoplastic disorders such as, for example, prostate cancer. Caveolin is an integral membrane protein and a principal component of caveolae. Caveolae are small invaginations at or near the plasma membrane of most smooth muscle cells and may function as a component of specific signal transduction pathways. Surprisingly, caveolin expression increases in metastatic human prostate cells as compared to human primary prostate tumors.

As caveolin expression correlates with metastasis, application of biological technologies designed to block the activity of caveolin or the function of caveolae may have therapeutic benefits for the treatment of neoplastic disorders such as human prostate tumors. Specific treatment approaches using caveolin may include the delivery of antisense or dominant negative caveolin sequences using expression or viral vectors; as well as the use of specific anti-caveolin antibodies. Additional approaches could also target the cavoeolae, but are not specifically based on caveolin function. Additional protein and non-protein components of caveolae could also be targeted for abrogation or the local or systemic administration of nutritional or biological agent may also be used. For example, caveolae are extremely rich in cholesterol and disruption or depletion of this molecule may alter the function of caveolae.

Methods for treating a neoplastic disorder comprising administering a pharmaceutically effective amount of composition containing a nucleic acid having a sequence identified according to the methods of this invention, its expression product or fragments of either are contemplated. The nucleic acid may be in the form of a sense or antisense single-stranded or double-stranded nucleic acid. The composition may be combined with a pharmaceutically acceptable carrier such as water, alcohols, salts, oils, fatty acids, saccharides, polysaccharides administered by injection, pulmonary absorption, topical application or delayed release. More than one carrier may be used together to create a pharmaceutical with desirable properties.

A kit or diagnostic aid for screening biological samples for detection of metastasis, neoplasia or kits comprise sequences isolated according to the methods of the invention and reagents and materials useful in such kits, such as, for example, buffers, salts, preservatives, and carriers, all of which are well known to those of ordinary skill in the art is also contemplated. Kits are useful for the analysis of tissues to screen those for the determination of normal, nonmalignant neoplastic or malignant cells. Kits may comprise additional reagents useful for the extraction of nucleic acids from a tissue sample. Reagents for analyzing the nucleic acid extracted from a tissue sample such as polymerase chain reaction reagents and Southern blots reagents may also be included.

The following experiments are offered to illustrate embodiments of the invention and should not be viewed as limiting the scope of the invention.

### Examples

### Example 1 Production of Mouse Prostate Reconstitution Tumors and Metastasis.

Mouse Urogenital Sinus (UGS) tissue was isolated from 17 day old mice embryos. Each isolated UGS was digested with 1% trypsin for three hours at 4°C. The trypsin was inactivated by the addition of fetal calf serum. UGS cells were digested with 0.125% collagenase for 1.5 hours, counted and mixed at the appropriate cell rations prior to infection with retrovirus in the presence of polybrene. Retroviruses used include Zipras/myc-9. Control experiments were performed using BAGα virus. After a two-hour infection, the infected cells were centrifuged and individual reconstitutions containing 1.5 x 10⁶ cells produced by resuspending the cells in rat tail collagen at a density of 6.0 x 10⁷ cells per ml. Aliquots of the infected UGS cells were placed in (DME) with 10% fetal calf serum overnight at 37°C, 5% CO₂.

The next morning each cell/collagen reconstitution was implanted under the renal capsule of an adult male +/+ animal. Reconstitutions were harvested from the mice five weeks later when they showed signs of obvious distress from the tumor burden. Metastasized tumors were isolated from the same mice at sites outside the renal capsule. Isolated tumors and metastasises were either stored in liquid nitrogen or in preservatives such as 10% buffered formalin.

Cell lines were derived from fresh tumors by mincing a small portion of the primary metastatic or nonmetastatic tumor and placing each in explant culture in Dulbecco's Modified Eagle Medium (DMEM) supplemented with 10% fetal calf serum. Cells which grow from each explant were propagated in DMEM and 10% fetal calf serum.

For histological analysis, a portion of a fresh tumor was fixed in 10% buffered formalin and embedded in paraffin for sectioning and staining with hematoxylin and eosin (H&E) or immunohistochemical staining. Immunohistochemical localization of cytokeratins was detected using polyclonal cytokeratin antiserum A575 (Dake Co.; Carpinteria, CA) and Vectastain™ ABC kit (Vector Laboratories; Burlingame, CA).

### Example 2 Isolation of C-DNA for DD-PCR.

Total cellular RNA was isolated by ultracentrifugation through cesium chloride. Briefly, up to one gram of cells from culture, tumors or organs was placed into 4 ml of ice-cold GIT buffer (4M guanidine isothiocyanate, 0.025 M sodium acetate, 0.1 M β-mercaptoethanol) and homogenized in a tissue homogenizer (Polytron or equivalent). The homogenate was carefully layered over 4 ml of 5.7 M CsCl, 0.024 M sodium acetate (1.8 g CsCl per ml) in a centrifuge tube. The layers were centrifuged at 35,000 RPM for 18 hours in a SW50.1 rotor. DNA was collected from the interface between the cushion and the supernatant, diluted two folds with water, added to 2.5 volumes of ethanol and spooled out on a glass rod. RNA that formed a pellet on the bottom of the CsCl layer was resuspended, and once extracted with an equal volume of phenol:chloroform (1:1), twice with chloroform and precipitated with ethanol and resuspended in diethylpyrocarbonate treated water. The concentration of DNA and RNA were be determined by absorption at 260 nanometers.

### Example 3 Differential Display Polymerase Chain Reaction.

mRNA isolated from primary tumors or metastasis was reverse transcribed with one of the primers and subjected to DD-PCR using the same primer as both the forward and reverse primer. A set of 24 primers comprising short oligonucleotides were used for both the reverse transcription of mRNA into c-DNA and for differential display polymerase chain reaction. The sequence of the primers used are shown in Table 1.

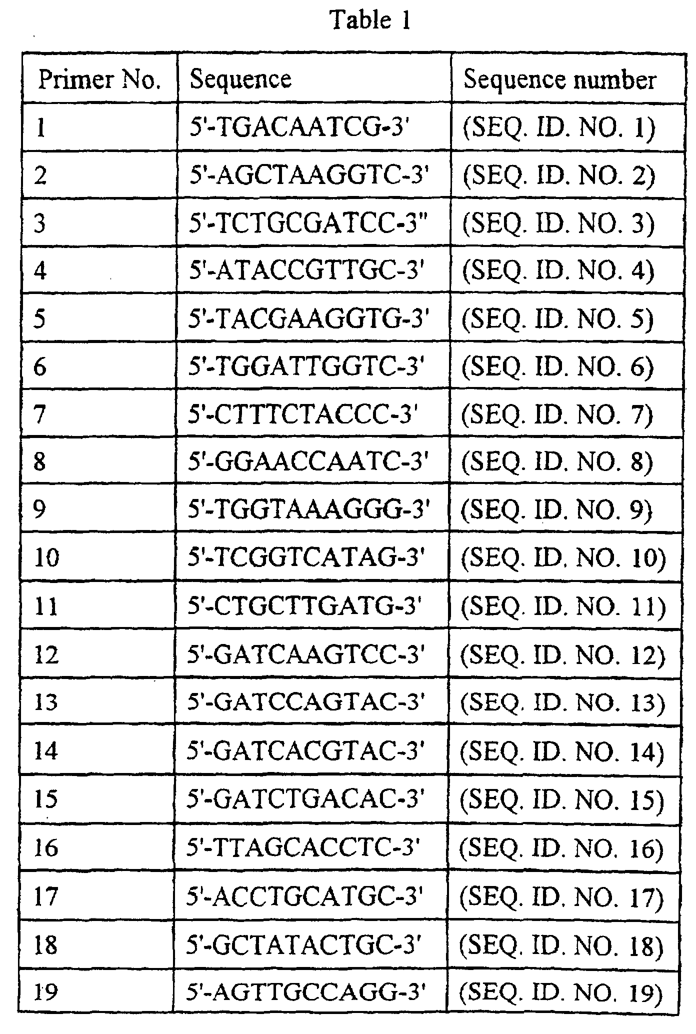

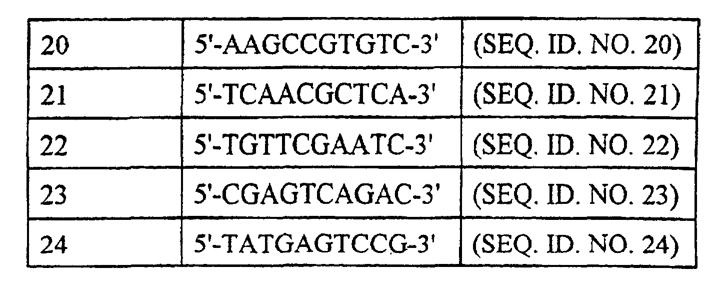

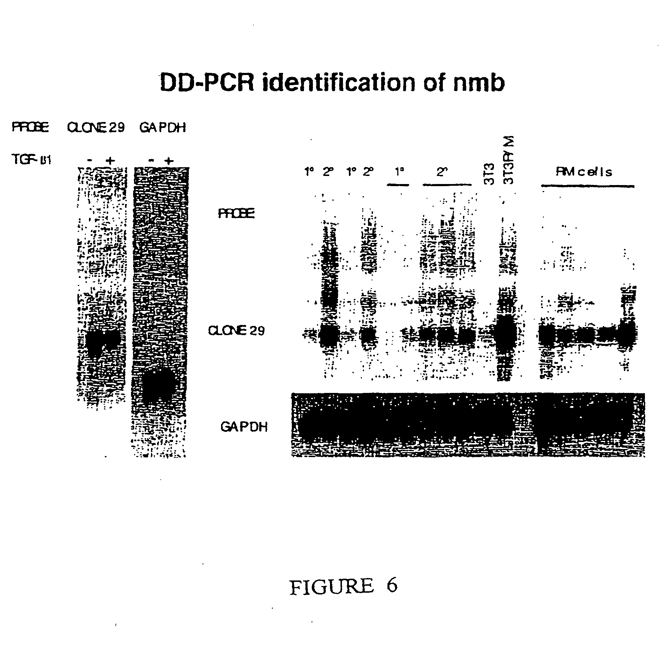

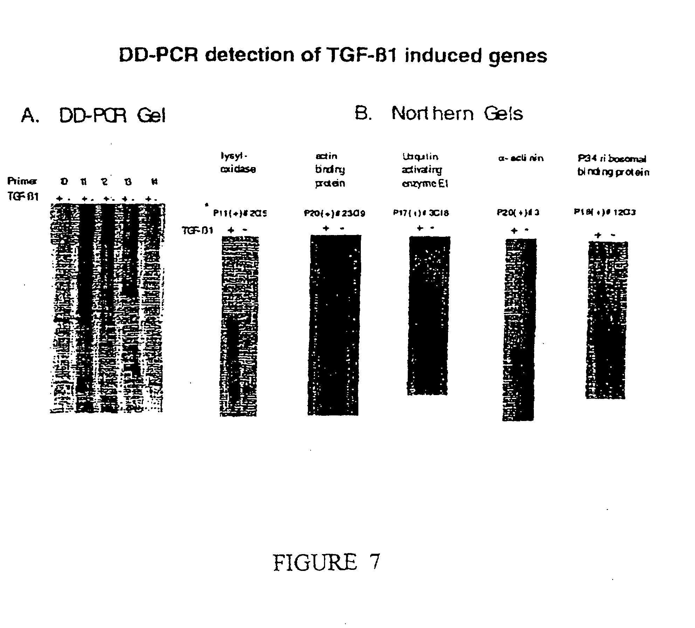

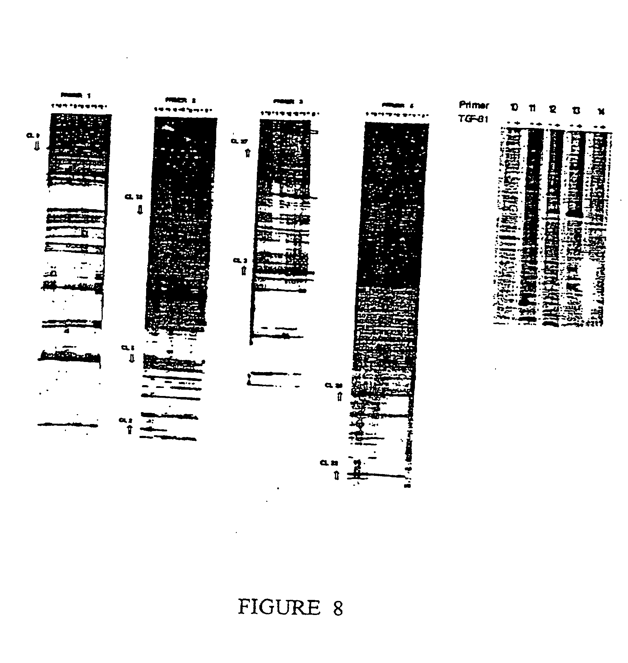

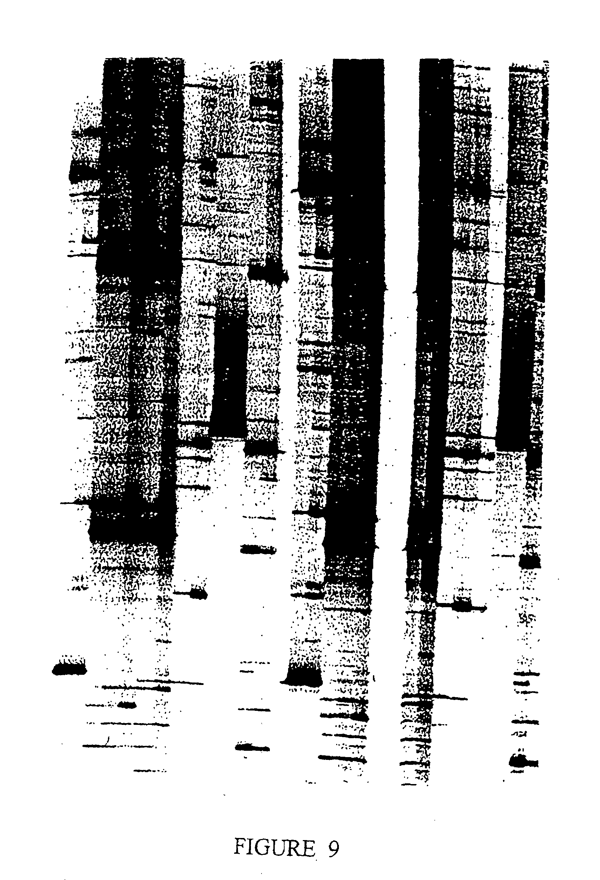

PCR was performed using standard conditions with 40 cycles of denaturation at 94°C for 40 seconds, annealing at 40°C for 2 minutes, and elongation at 72°C for 35 seconds. After PCR, the products were analyzed with non-denaturing polyacrylamide gel electrophoresis (PAGE) at 12 watts for 15 hours. Bands which differed between test and control samples were eluted from the gel, subjected to reamplification by PCR and cloned. Polyacrylamide gel electrophoresis of DD-PCRs, and the accompanying RNA blot analysis showing the isolation of sequences with substantial similarity to nmb and TGF-β is shown in Figure 6 and Figure 7 respectively. Additional sequences isolated by this method show substantial similarity to lysyl oxidase, actin binding protein, ubiquitin activating enzyme E1, α-actinin, and P34 ribosomal binding protein sequence (Figure 8). Differential expression of caveolin was demonstrated by DD-PCR followed by PAGE (Figure 9).

### Example 4 p53 Allelotype Determination.

The *p53* allelotype of a cell sample was determined by PCR. Briefly, nucleic acid is extracted from a tissue sample or a cell culture sample. An aliquot of nucleic acids in placed in 45 µl aliquot of a master mix which contained a final concentration of 0.2 mM of each dATP, dTTP, dGTP, dCTP, 1.5 mM MgCl₂, 0.5 unit Taq polymerase, 0.05 µM of each of two primers set specific for the normal wildtype allele of *p53* (5'-GTGTTTCATTAGTTCCCCACCTTGAC-3', SEQ. ID NO. 25; 5'-AGAGCAAGAATAAGTCAGAAGCCG-3', SEQ. ID NO. 26). A control set of primers specific for the fibroblast growth factor-7 gene was used to monitor the polymerase chain reaction experiment (5'-ACAGACCGTGCTTCCACCTCGTC-3', SEQ. ID NO. 27; 5'-CCTCATCTCCTGGGTCCCTTTCA-3', SEQ. ID NO.28). One µl of the reaction from the first round of PCR was used as the starting material for a second round of PCR using a second set of wildtype *p53* specific primer (5'-GTCCGCGCCATGGCCATATA-3', SEQ. ID NO. 29; 5'-ATGGGAGGCTGCCAGTCCTAACCC-3', SEQ. ID NO. 30). This second round of PCR was also monitored using a control set of primers specific for the fibroblast growth factor-7 (5'-ACAGACCGTGCTTCCACCTCGTC-3', SEQ. ID NO 27; 5'-CCTCATCTCCTGGGTCCCTTTCA-3', SEQ. ID NO 28).

After PCR the products were analyzed with non-denaturing polyacrylamide gel electrophoresis (PAGE) at 12 watts for 15 hours. Bands which differed between test and control were eluted from the gel, subjected to reamplification by PCR and cloned.

### Example 5 Induction of cell lines with TGF-β1 Influence Cellular Gene Expression.

1481-PA cells were grown overnight in DME supplemented with 10% fetal calf serum overnight at 37°C, and 5% CO₂. Induction was performed by treatment with *TGF-β1* at a concentration of 2 nanograms per ml. The treated cells were returned to the incubator and cultured for 12 hours. After induction, cells were washed in phosphate buffered saline and harvested and concentrated by centrifugation.

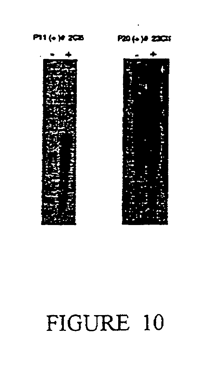

RNA was extracted from treated and untreated cells and subjected to DD-PCR. Differentially expressed bands detected by DD-PCR were cloned and differential expressions were confirmed using RNA blots (Figure 10). Subsequent cloning and sequencing identified the bands as ABP280 or filamin.

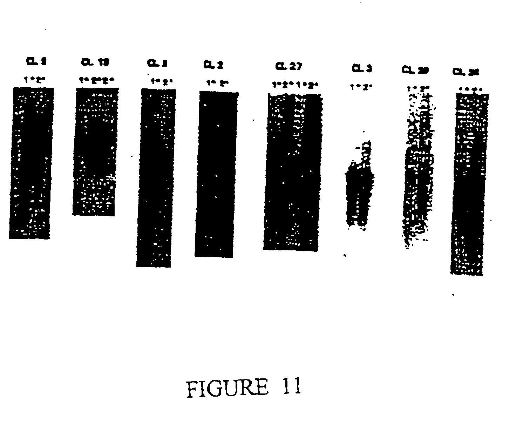

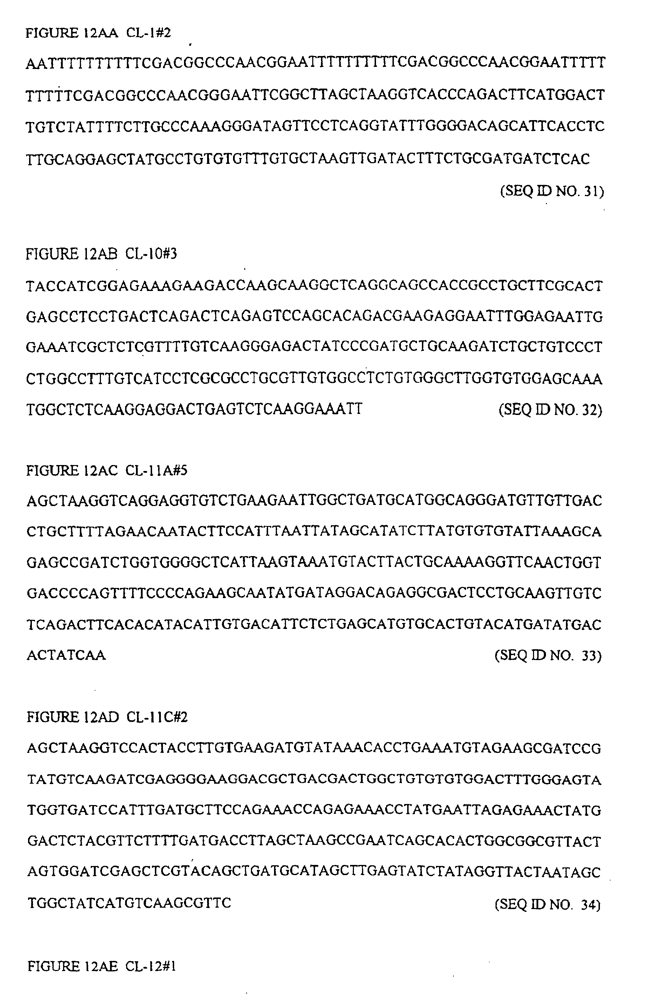

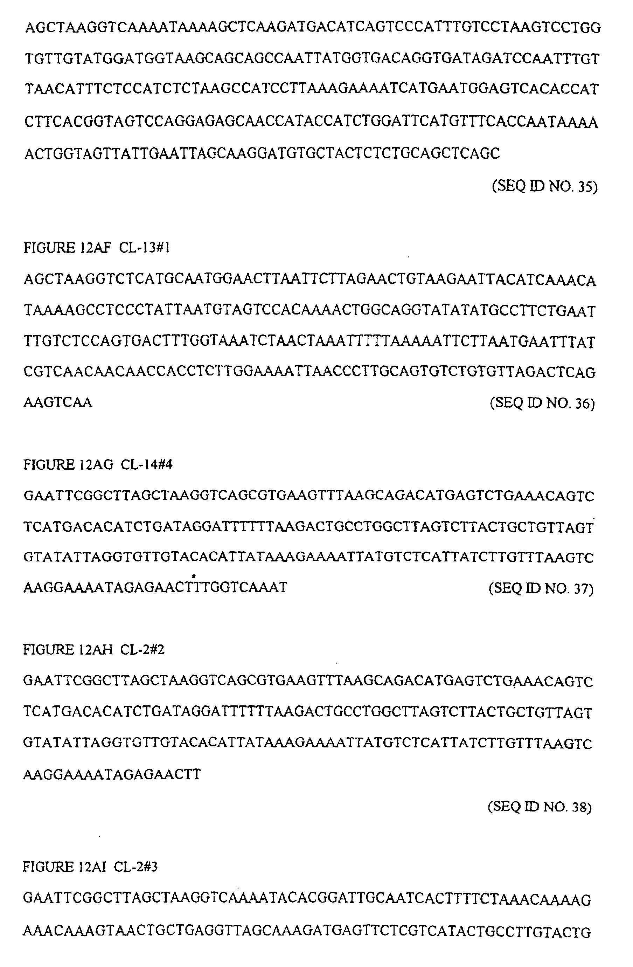

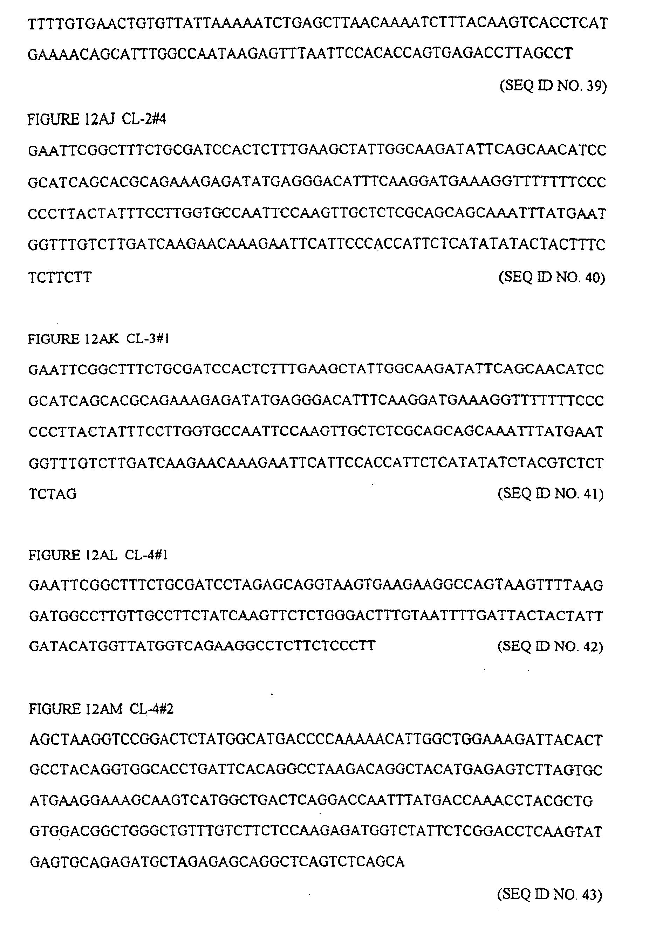

One gene isolated showed differential expression in cells induced by *TGF-β* (Figure 11, clone 29), while a control probe on the same cell line showed no difference in expression levels (Figure 11, GAPDH).

### Example 6 Metastatic Sequences Isolated.

Using the methods of Examples 1, 2, 3, 4, and 5, a plurality of metastatic sequences were isolated and sequenced. The expression of the metastatic sequences in primary cells and in metastatic cells were determined using RNA blots. The nucleic acid sequences of other isolated sequences are listed in Figure 12. Sequence analysis and expression analysis was performed on the isolated cloned and the results of these studies are summarized in Figure 13.

### Example 7 Caveolin Immunoassay in Human Prostate Cancers.

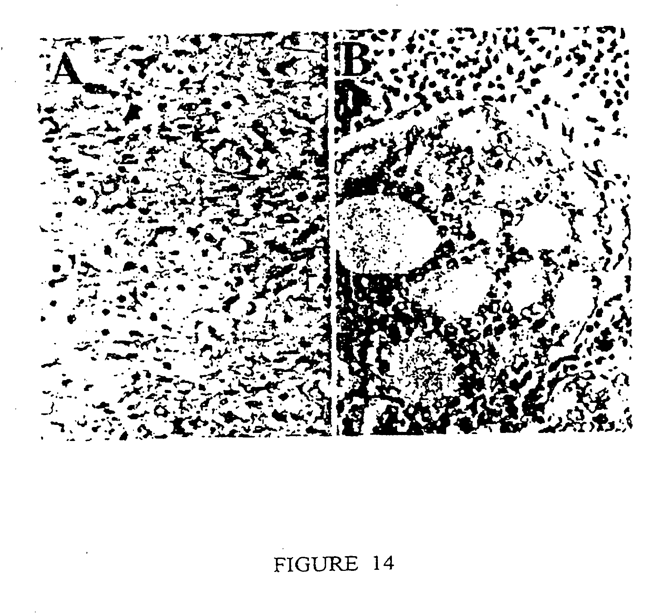

Primary site human prostate tumors and metastases were isolated and analyzed for caveolin expression by immunoassay. The results of the assay is shown in Table 3. Metastases shows higher levels of caveolin proteins in metastases than in primary tumors. Immunohistology of tissue sections reveals both elevated levels and distinct distribution of caveolin protein in metastatic human prostate when compared to a primary human prostate tumor (Figure 14).

**Table 3**

| Patients | Primary-site | Metastases in lymph node |
|---|---|---|
| 1 | + | ++ |
| 2 | ++ | +++ |
| 3 | ++ | +++ |
| 4 | ++ | ++ |
| 5 | + | + |
| 6 | ++ | ++ |
| 7 | ++ | +++ |
| 8 | + | + |
| 9 | - | - |
| 10 | + | + |
| 11 | + | + |
| 12 | ++ | ++ |
| 13 | + | + |
| 14 | ++ | +++ |

Other embodiments and uses of the invention will be apparent to those skilled in the art from consideration of the specification and practice of the invention disclosed herein.

## Claims

1. A method for identifying a metastatic sequence comprising the steps of:
a) transfecting an oncogenic sequence into a mammalian cell in-vitro to form a population of transfected cells;
b) introducing transfected cells to a primary site of a non-human host mammal;
c) maintaining said mammal for a period of time sufficient to develop a metastasis at a secondary site;
d) amplifying expressed sequences of the transfected cells and expressed sequences of the metastasis by differential-display PCR; and
e) comparing the amplified expressed sequences of the transfected cells with the amplified expressed sequences of the metastasis to identify the metastatic sequence.

2. The method of Claim 1 wherein the mammalian cell is transfected by calcium phosphate transfection, viral transduction, lipofection, dextran sulfate transfection or electroporation.

3. The method of Claim 1 or Claim 2 wherein the oncogenic sequence is a sequence of the gene that expresses the oncoproteins p21, p34, mutant p53, myc, ras or src.

4. The method of Claim 1 or Claim 2 wherein the oncogenic sequence is a sequence that enhances metastatic potential.

5. The method of Claim 4 wherein the metastatic sequence is a sequence of the gene that encodes cyclin D1, caveolin or TGF-β1.

6. The method of Claim 1 wherein the metastatic sequence identified is specifically expressed in non-metastatic cells.

7. The method of Claim 1 or Claim 2 wherein the oncogenic sequence is a gene ablation sequence specific for the gene that expresses the protein TGF-β1, cyclin D1, p21, p34, p35, lysyl oxidase, caveolin, actin binding protein, ubiquitin activating enzyme El, nmb or α actinin 3.

8. The method of any of Claims 1 to 7 wherein the mammalian cell is treated before or after transfection with
(i) an agent that alters gene expression; or
(ii) benzanthracene, dimethyl benzanthracene or 5-azacytidine.

9. The method of any of Claims 1 to 8 wherein the mammalian cell is
(i) a primary or established cell line; and/or
(ii) derived from lymphatic tissue, hematopoietic cells, reproductive tissues or urogenital sinus tissue and preferably from urogenital sinus tissue; and/or
(iii) a fetal cell.

10. The method of any of Claims 1 to 9 wherein the mammalian cell is derived from the same species as the host mammal and/or the mammalian cell and the host mammal are histocompatible.

11. The method of any of Claims 1 to 9 wherein the mammalian cell and the host mammal are syngeneic or xenogeneic.

12. The method of any of Claims 1 to 11 wherein the transfected cells are maintained *in vivo* or *in vitro* for a period of time prior to introduction of the transfected cells to the host mammal.

13. The method of any of Claims 1 to 12 wherein the expressed sequences of the transfected cells are obtained from
(i) cells at the primary site of the host mammal; or
(ii) a cell line of immortalized transfected cells.

14. The method of any of Claims 1 to 13 wherein the transfected cells are introduced to the primary site by subcutaneous implantation.

15. The method of any of Claims I to 14 wherein the host mammal :
(i) is a mouse, a rabbit or a primate; and/or
(ii) is an immunocompromised or transgenic host mammal; and/or
(ii) has reduced expression of *TGF-β*.

16. The method of any of Claims 1 to 15 wherein the primary site is the renal capsule, the prostate or the testis and/or the secondary site is selected from the lung, kidney, liver, lymph nodes, brain, bone, testis, spleen, ovaries and mammary.

17. The method of any of Claims 1 to 16 wherein the differential display PCR is performed with an anchor primer and a variable primer.

18. The method of Claim 17 wherein the anchor primer comprises a polythymidine sequence and a dinucleotide sequence connected to a 3'-terminus.

19. The method of Claim 17 or Claim 18 wherein the anchor primer comprises a polythymidine sequence which comprises between about 5 and about 30 thymidines and optionally the dinucleotide sequence is selected from AA, AG, AC, AT, GA, GG, GC, GT, CA, CG, CC and CT.

20. The method of any of Claims 17 to 19 wherein the anchor primer or the variable primer comprises a detectable moiety selected from radioactive moieties, phosphorescent moieties, magnetic moieties, luminescent moieties and conjugatable moieties.

21. The method of any of Claims 17 to 20 wherein the anchor primer and the variable primer have a common sequence.

22. The method of any of Claims 1 to 21 further comprising the step of treating the host mammal with a metastatic agent, the metastatic agent optionally being a retinoid.

23. The method of any of Claims 1 to 22 wherein step (e) comprises determining the nucleotide sequence or expression product of the metastatic sequence.

24. A method for identifying a metastatic sequence comprising the steps of:
a) introducing mammalian cells pretreated with a metastatic agent to a primary site of a non-human host mammal, to form a population of cells predisposed to metastasis;
b) maintaining said mammal for a period of time sufficient to develop a metastasis at a secondary site;
c) amplifying (i) expressed sequences of the pretreated cells or non-pretreated cells and (ii) expressed sequences of the metastasis, by differential-display PCR; and
d) comparing (i) the amplified expressed sequences of the pretreated cells or non-pretreated cells with (ii) the amplified expressed sequences of the metastasis to identify the metastatic sequence.

25. The method of Claim 24 wherein the metastatic agent is a chemical compound, a nucleic acid, a protein or a combination thereof.

26. The method of Claim 25 wherein the chemical compound is a benzanthracene, dimethyl benzanthracene or 5-azacytidine.

27. The method of Claim 25 wherein the metastatic agent is TGF-β1, Cyclin D1, p21, p34, mutant p53, lysyl oxidase, caveolin, actin binding protein, ubiquitin activating enzyme El, nmb, α-actinin 3, myc or ras.

28. The method of any of Claims 24 to 27 wherein the cells of the primary or secondary site are treated with a genotoxic agent, wherein optionally the genotoxic agent is benzathracene, dimethylbenzathracene or 5-azacytidine.

29. The method of Claim 28 wherein the metastatic agent and the genotoxic agent are the same.

30. The method of any of Claims 24 to 29 wherein the mammalian cell is transfected with an oncogenic sequence before or after pretreatment.

31. The method of any of Claims 24 to 30, wherein the mammalian cells are
(i) a primary or established cell line; and/or
(ii) derived from lymphatic tissue, hematopoietic cells, reproductive tissues or urogenital sinus tissue and preferably from urogenital sinus tissue; and/or
(iii) fetal cells.

32. The method of any of claims 24 to 31, wherein the mammalian cells are derived from the same species as the host mammal and/or the mammalian cell and the host mammal are histocompatible.

33. The method of any of Claims 24 to 32, wherein the primary site is the renal capsule, the prostate or the testis and/or the secondary site is selected from the lung, kidney, liver, lymph nodes, brain, bone, testis, spleen, ovaries and mammary.

34. The method of any of Claims 24 to 33, wherein the differential display PCR is performed with an anchor primer and a variable primer.

35. The method of Claim 34, wherein the anchor primer comprises a polythymidine sequence and a dinucleotide sequence connected to a 3'-terminus.

36. The method of Claim 34 or Claim 35, wherein the anchor primer comprises a polythymidine sequence which comprises between about 5 and about 30 thymidines and optionally the dinucleotide sequence is selected from AA, AG, AC, AT, GA, GG, GC, GT, CA, CG, CC and CT.

37. The method of any of Claims 34 to 36, wherein the anchor primer or the variable primer comprises a detectable moiety selected from radioactive moieties, phosphorescent moieties, magnetic moieties, luminescent moieties and conjugatable moieties.

38. The method of any of Claims 34 to 37, wherein the anchor primer and the variable primer have a common sequence.

39. The method of any of claims 24 to 38, wherein the mammalian cells are derived from a transgenic animal.

## Patentansprüche

1. Ein Verfahren zur Identifizierung einer metastatischen Sequenz, umfassend die Schritte:
a) Transfizieren einer onkogenen Sequenz *in vitro* in eine Säugerzelle, um eine Population transfizierter Zellen zu bilden;
b) Einführen transfizierter Zellen in eine erste Stelle eines nicht-humanen Wirtssäugers;
c) Aufrechterhalten des Säugers für einen Zeitraum, der ausreichend ist, um eine Metastase an einer zweiten Stelle zu entwickeln;
d) Amplifizieren der exprimierten Sequenzen der transfizierten Zellen und exprimierten Sequenzen der Metastase durch *Differential-Display* PCR; und
e) Vergleichen der amplifizierten exprimierten Sequenzen der transfizierten Zellen mit den amplifizierten exprimierten Sequenzen der Metastase, um die metastatische Sequenz zu identifizieren.

2. Verfahren gemäß Anspruch 1, wobei die Säugerzelle durch Kalzium-Phosphat-Transfektion, virale Transduktion, Lipofektion, Dextran-Sulfat-Transfektion oder Elektroporation transfiziert wird.

3. Verfahren gemäß Anspruch 1 oder Anspruch 2, wobei die onkogene Sequenz eine Sequenz des Gens ist, das die Onkoproteine p21, p34, Mutanten p53, myc, ras oder src exprimiert.

4. Verfahren gemäß Anspruch 1 oder Anspruch 2, wobei die onkogene Sequenz eine Sequenz ist, die das metastatische Potential verstärkt.

5. Verfahren gemäß Anspruch 4, wobei die metastatische Sequenz die Sequenz eines Gens ist, das Cyclin D1, Caveolin oder TGF-β1 kodiert.

6. Verfahren gemäß Anspruch 1, wobei die identifizierte metastatische Sequenz spezifisch in nicht-metastatischen Zellen exprimiert wird.

7. Verfahren gemäß Anspruch 1 oder Anspruch 2, wobei die onkogene Sequenz eine Gen-Ablations-Sequenz ist, die spezifisch für das Gen ist, das das Protein TGF-β1, Cyclin D1, p21, p34, p35, Lysyloxidase, Caveolin, Actin-bindendes Protein, Ubiquitin-aktivierendes Enzym E1, nmb oder α-Actinin 3 exprimiert.

8. Verfahren gemäß einem der Ansprüche 1 bis 7, wobei die Säugerzelle vor oder nach der Transfektion behandelt wird mit
(i) einem Mittel, das die Genexpression ändert; oder
(ii) Benzanthracen, Dimethyl-benzanthracene.

9. Verfahren gemäß einem der Ansprüche 1 bis 8, wobei die Säugerzelle
(i) eine primäre oder etablierte Zelllinie; und / oder
(ii) abgeleitet von lymphatischem Gewebe, hematopoetischen Zellen, reproduktiven Geweben oder Urogenital-Sinus-Gewebe und vorzugsweise von Urogenital-Sinus Gewebe; und / oder
(iii) eine fötale Zelle ist.

10. Verfahren gemäß einem der Ansprüche 1 bis 9, wobei Säugerzelle abgeleitet ist von der gleichen Spezies, wie der Wirtssäuger und / oder die Säugerzelle und der Wirtssäuger histokompatibel sind.

11. Verfahren gemäß einem der Ansprüche 1 bis 9, wobei die Säugerzelle und der Wirtssäuger synergen oder xenogen sind.

12. Verfahren gemäß einem der Ansprüche 1 bis 11, wobei die transfizierten Zellen *in* vivo oder *in vitro* für einen Zeitraum vor der Einführung der transfizierten Zellen in den Wirtssäuger aufrechterhalten werden.

13. Verfahren gemäß einem der Ansprüche 1 bis 12, wobei die exprimierten Sequenzen der transfizierten Zellen erhalten werden von:
(i) Zellen an der ersten Stelle in dem Wirtssäuger; oder
(ii) einer Zelllinie von immortalisierten transfizierten Zellen.

14. Verfahren gemäß einem der Ansprüche 1 bis 13, wobei die transfizierten Zellen in die erste Stelle durch subkutane Implantation eingeführt werden.

15. Verfahren gemäß einem der Ansprüche 1 bis 14, wobei der Wirtssäuger.
(i) eine Maus, ein Kaninchen oder ein Primat ist; und / oder
(ii) ein immungeschwächter oder transgener Wirtssäuger ist; und / oder
(iii) reduzierte Expression von *TGF-β* hat.

16. Verfahren gemäß einem der Ansprüche 1 bis 15, wobei die erste Stelle die Bindegewebskapsel der Niere, die Prostata oder der Hoden ist und / oder die zweite Stelle ausgewählt ist aus der Lunge, Niere, Leber, Lymphknoten, Gehirn, Knochen, Hoden, Milz, Eierstöcken und Brust.

17. Verfahren gemäß einem der Ansprüche 1 bis 16, wobei die *Differential-Display* PCR mit einem Anker-Primer und einem variablen Primer durchgeführt wird.

18. Verfahren gemäß Anspruch 17, wobei der Anker-Primer eine Polythymidin-Sequenz und eine Dinukleotidsequenz, verbunden mit einem 3'-Terminus, umfasst.

19. Verfahren gemäß Anspruch 17 oder Anspruch 18, wobei der Anker-Primer eine Polythymidin-Sequenz umfasst, die zwischen etwa 5 und etwa 30 Thymidinen umfasst und optional ist die Dinukleotidsequenz ausgewählt aus AA, AG, AC, AT, GA, GG, GC, GT, CA, CG, CC und CT.

20. Verfahren gemäß einem der Ansprüche 17 bis 19, wobei der Anker-Primer oder der variable Primer eine nachweisbare Gruppe, ausgewählt aus radioaktiven Gruppen, Phosphoreszenz-Gruppen, magnetischen Gruppen, Lumineszenz-Gruppen und konjugierbaren Gruppen, umfasst.

21. Verfahren gemäß einem der Ansprüche 17 bis 20, wobei der Anker-Primer und der variable Primer eine gemeinsame Sequenz haben.

22. Verfahren gemäß einem der Ansprüche 1 bis 21, weiterhin umfassend den Schritt der Behandlung des Wirtssäugers mit einem metastatischen Mittel, wobei das metastatische Mittel optional ein Retinoid ist.

23. Verfahren gemäß einem der Ansprüche 1 bis 22, wobei Schritt (e) die Bestimmung der Nukleotidsequenz oder des Expressionsprodukts der metastatischen Sequenz umfasst.

24. Verfahren zu Identifizierung einer metastatischen Sequenz, umfassend die Schritte:
a) Einführen von Säugerzellen, die mit einem metastatischen Mittel vorbehandelt sind, in eine erste Stelle eines nicht-humanen Wirtssäugers, um eine Population von Zellen zu bilden, die prädisponiert für Metastase sind;
b) Aufrechterhalten des Säugers für einen Zeitraum, der ausreichend ist, um eine Metastase an einer zweiten Stelle zu entwickeln;
c) Amplifikation (i) der exprimierten Sequenzen der vorbehandelten Zellen oder nicht-vorbehandelten Zellen und (ii) der exprimierten Sequenzen der Metastase, durch *Differential-Display* PCR; und
d) Vergleichen (i) der amplifizierten exprimierten Sequenzen der vorbehandelten Zellen oder nicht-vorbehandelten Zellen mit (ii) den amplifizierten exprimierten Sequenzen der Metastase, um die metastatischen Sequenz zu Identifizieren.

25. Verfahren gemäß Anspruch 24, wobei das metastatische Mittel eine chemische Verbindung, eine Nukleinsäure, ein Protein oder eine Kombination davon ist.

26. Verfahren gemäß Anspruch 25, wobei die chemische Verbindung ein Benzanthracen, Dimethyl-Benzanthracen oder 5-Azacytidin ist.

27. Verfahren gemäß Anspruch 25, wobei das metastatische Mittel TGF-β, Cyclin D1, p21, p34, Mutanten p53, Lysyloxidase, Caveolin, Actin-bindendes Protein, Ubiquitin-aktivierendes Enzym E1, nmb, α-Actinin 3, myc oder ras ist.

28. Verfahren gemäß einem der Ansprüche 24 bis 27, wobei die Zellen der ersten oder der zweiten Stelle mit einem genotoxischen Mittel behandelt werden, wobei das genotoxische Mittel optional Benzanthracen, Dimethyl-Benzanthracen oder 5-Azacytidin ist.

29. Verfahren gemäß Anspruch 28, wobei das metastatische Mittel und das genotoxische Mittel die gleichen sind.

30. Verfahren gemäß einem der Ansprüche 24 bis 29, wobei die Säugerzelle mit einer onkogenen Sequenz, vor oder nach der Vorbehandlung, transfiziert wird.

31. Verfahren gemäß einem der Ansprüche 24 bis 30, wobei die Säugerzellen
(i) eine primäre oder etablierte Zelllinie ist; und / oder
(ii) abgeleitet ist von lymphatischem Gewebe, hematopoetischen Zellen, reproduktiven Geweben oder Urogenital-Sinus-Gewebe und vorzugsweise von Urogenital-Sinus-Gewebe; und/oder
(iii) fötale Zellen sind.

32. Verfahren gemäß einem der Ansprüche 24 bis 31, wobei die Säugerzellen abgeleitet sind von der gleichen Spezies wie der Wirtssäuger und / oder die Säugerzelle und der Wirtssäuger histokompatibel sind.

33. Verfahren gemäß einem der Ansprüche 24 bis 32, wobei die erste Stelle die Bindegewebskapsel der Niere, die Prostata oder der Hoden ist und / oder die zweite Stelle ausgewählt ist aus der Lunge, Niere, Leber, Lymphknoten, Gehirn, Knochen, Hoden, Milz, Eierstöcken und Brust.

34. Verfahren gemäß einem der Ansprüche 24 bis 33, wobei die *Differential-Display* PCR mit einem Anker-Primer und einem variablen Primer durchgeführt wird.

35. Verfahren gemäß Anspruch 34, wobei der Anker-Primer eine Polythymidin-Sequenz und eine Dinukleotidsequenz, verbunden mit einem 3'-Terminus, umfasst.

36. Verfahren gemäß Anspruch 34 oder Anspruch 35, wobei der Anker-Primer eine Polythymidin-Sequenz umfasst, die zwischen etwa 5 und etwa 30 Thymidinen umfasst und optional ist die Dinukleotidsequenz ausgewählt aus AA, AG, AC, AT, GA, GG, GC, GT, CA, CG, CC und CT.

37. Verfahren gemäß einem der Ansprüche 34 bis 36, wobei der Anker-Primer oder der variable Primer eine nachweisbare Gruppe, ausgewählt aus radioaktiven Gruppen, Phosphoreszenz-Gruppen, magnetischen Gruppen, Lumineszenz-Gruppen und konjugierbaren Gruppen, umfasst.

38. Verfahren gemäß einem der Ansprüche 34 bis 37, wobei der Anker-Primer und der variable Primer eine gemeinsame Sequenz haben.

39. Verfahren gemäß einem der Ansprüche 24 bis 38, wobei die Säugerzellen von einem transgenen Tier abgeleitet sind.

## Revendications

1. Procédé pour identifier une séquence métastatique comprenant les étapes consistant à :
a) transfecter une séquence oncogène dans une cellule de mammifère in vitro pour former une population de cellules transfectées ;
b) introduire les cellules transfectées dans un site primaire d'un mammifère hôte non humain ;
c) maintenir ledit mammifère pendant une période suffisante pour développer une métastase au niveau d'un site secondaire ;
d) amplifier les séquences exprimées des cellules transfectées et les séquences exprimées de la métastase par amplification en chaîne par polymérase (PCR, polymerase chain reaction) par affichage différentiel ; et
e) comparer les séquences exprimées amplifiées des cellules transfectées avec les séquences exprimées amplifiées de la métastase pour identifier la séquence métastatique.

2. Procédé selon la revendication 1, dans lequel la cellule de mammifère est transfectée par une transfection au phosphate de calcium, une transduction virale, une lipofection, une transfection au sulfate de dextrane ou une électroporation.

3. Procédé selon la revendication 1 ou la revendication 2, dans lequel la séquence oncogène est une séquence du gène qui exprime les oncoprotéines p21, p34, p53 mutante, myc, ras ou src.

4. Procédé selon la revendication 1 ou la revendication 2, dans lequel la séquence oncogène est une séquence qui augmente le potentiel métastatique.

5. Procédé selon la revendication 4, dans lequel la séquence métastatique est une séquence du gène qui code pour la cycline D1, la cavéoline ou le TGF-β1.

6. Procédé selon la revendication 1, dans lequel la séquence métastatique identifiée est spécifiquement exprimée dans des cellules non métastatiques.

7. Procédé selon la revendication 1 ou la revendication 2, dans lequel la séquence oncogène est une séquence d'ablation de gène spécifique au gène qui exprime la protéine TGF-β1, la cycline D1, la p21, la p34, la p35, l'oxydase de lysyle, la cavéoline, la protéine de liaison d'actine, l'enzyme E1 d'activation d'ubiquitine, la nmb ou l'α actinine 3.

8. Procédé selon l'une quelconque des revendications 1 à 7, dans lequel la cellule de mammifère est traitée avant ou après la transfection avec
(i) un agent qui altère l'expression du gène ; ou
(ii) du benzanthracène, du diméthylbenzanthracène ou de la 5-azacytidine.

9. Procédé selon l'une quelconque des revendications 1 à 8, dans lequel la cellule de mammifère est
(i) une lignée cellulaire primaire ou établie ; et/ou
(ii) dérivée de tissu lymphatique, de cellules hématopoïétiques, de tissus de l'appareil reproducteur ou de tissu du sinus urogénital et de préférence de tissu du sinus urogénital ; et/ou
(iii) une cellule foetale.

10. Procédé selon l'une quelconque des revendications 1 à 9, dans lequel la cellule de mammifère est dérivée de la même espèce que le mammifère hôte et/ou la cellule de mammifère et le mammifère hôte sont histocompatibles.

11. Procédé selon l'une quelconque des revendications 1 à 9, dans lequel la cellule de mammifère et le mammifère hôte sont syngéniques ou xénogéniques.

12. Procédé selon l'une quelconque des revendications 1 à 11, dans lequel les cellules transfectées sont maintenues *in vivo* ou *in vitro* pendant une certaine période avant l'introduction des cellules transfectées dans le mammifère hôte.

13. Procédé selon l'une quelconque des revendications 1 à 12, dans lequel les séquences exprimées des cellules transfectées sont obtenues à partir de
(i) cellules au niveau du site primaire du mammifère hôte ; ou
(ii) une lignée cellulaire de cellules transfectées immortalisées.

14. Procédé selon l'une quelconque des revendications 1 à 13, dans lequel les cellules transfectées sont introduites dans le site primaire par une implantation sous-cutanée.

15. Procédé selon l'une quelconque des revendications 1 à 14, dans lequel le mammifère hôte :
(i) est une souris, un lapin ou un primate ; et/ou
(ii) est un mammifère hôte fragilisé d'un point de vue immunitaire ou transgénique ; et/ou
(ii) a une expression réduite de *TGF-β*.

16. Procédé selon l'une quelconque des revendications 1 à 15, dans lequel le site primaire est la capsule rénale, la prostate ou les testicules et/ou le site secondaire est choisi parmi les poumons, les reins, le foie, les ganglions lymphatiques, le cerveau, les os, les testicules, la rate, les ovaires et les glandes mammaires.

17. Procédé selon l'une quelconque des revendications 1 à 16, dans lequel l'amplification en chaîne par polymérase (PCR, polymerase chain reaction) par affichage différentiel est réalisée avec une amorce d'ancrage et une amorce variable.

18. Procédé selon la revendication 17, dans lequel l'amorce d'ancrage comprend une séquence de polythymidine et une séquence de dinucléotide connectée à un terminus-3'.

19. Procédé selon la revendication 17 ou la revendication 18, dans lequel l'amorce d'ancrage comprend une séquence de polythymidine qui comprend entre environ 5 et environ 30 thymidines et optionnellement la séquence de dinucléotide est choisie parmi AA, AG, AC, AT, GA, GG, GC, GT, CA, CG, CC et CT.

20. Procédé selon l'une quelconque des revendications 17 à 19, dans lequel l'amorce d'ancrage ou l'amorce variable comprend un groupe fonctionnel détectable choisi parmi les groupes fonctionnels radioactifs, les groupes fonctionnels phosphorescents, les groupes fonctionnels magnétiques, les groupes fonctionnels luminescents et les groupes fonctionnels conjugables.

21. Procédé selon l'une quelconque des revendications 17 à 20, dans lequel l'amorce d'ancrage ou l'amorce variable ont une séquence commune.

22. Procédé selon l'une quelconque des revendications 1 à 21 comprenant en outre l'étape consistant à traiter le mammifère hôte avec un agent métastatique, l'agent métastatique étant optionnellement un rétinoïde.

23. Procédé selon l'une quelconque des revendications 1 à 22, dans lequel l'étape (e) comprenant l'étape consistant à déterminer la séquence de nucléotide ou le produit d'expression de la séquence métastatique.

24. Procédé pour identifier une séquence métastatique comprenant les étapes consistant à :
a) introduire les cellules de mammifères pré-traitées avec un agent métastatique dans un site primaire d'un mammifère hôte non humain, pour former une population de cellules prédisposées à la métastase ;
b) maintenir ledit mammifère pendant une période suffisante pour développer une métastase au niveau d'un site secondaire ;
c) amplifier (i) les séquences exprimées des cellules pré-traitées ou des cellules non pré-traitées et (ii) les séquences exprimées de la métastase par amplification en chaîne par polymérase (PCR, polymerase chain reaction) par affichage différentiel ; et
d) comparer (i) les séquences exprimées amplifiées des cellules pré-traitées ou des cellules non pré-traitées avec (ii) les séquences exprimées amplifiées de la métastase pour identifier la séquence métastatique.

25. Procédé selon la revendication 24, dans lequel l'agent métastatique est un composé chimique, un acide nucléique, une protéine ou une combinaison de ceux-ci.

26. Procédé selon la revendication 25, dans lequel le composé chimique est du benzanthracène, du diméthylbenzanthracène ou de la 5-azacytidine.

27. Procédé selon la revendication 25, dans lequel l'agent métastatique est le TGF-β1, la cycline D1, la p21, la p34, la p53 mutante, l'oxydase de lysyle, la cavéoline, la protéine de liaison d'actine, l'enzyme E1 d'activation d'ubiquitine, la nmb, l'α actinine 3, la myc ou la ras.

28. Procédé selon l'une quelconque des revendications 24 à 27, dans lequel les cellules du site primaire ou secondaire sont traitées avec un agent génotoxique, dans lequel l'agent génotoxique est optionnellement du benzanthracène, du diméthylbenzanthracène ou de la 5-azacytidine.

29. Procédé selon la revendication 28, dans lequel l'agent métastatique et l'agent génotoxique sont identiques.

30. Procédé selon l'une quelconque des revendications 24 à 29, dans lequel la cellule de mammifère est transfectée avec une séquence oncogène avant ou après le pré-traitement.

31. Procédé selon l'une quelconque des revendications 24 à 30, dans lequel les cellules de mammifère sont
(i) une lignée cellulaire primaire ou établie ; et/ou
(ii) dérivées de tissu lymphatique, de cellules hématopoïétiques, de tissus de l'appareil reproducteur ou de tissu de sinus urogénital et de préférence de tissu de sinus urogénital ; et/ou
(iii) des cellules foetales.

32. Procédé selon l'une quelconque des revendications 24 à 31, dans lequel les cellules de mammifère sont dérivées de la même espèce que le mammifère hôte et/ou la cellule de mammifère et le mammifère hôte sont histocompatibles.

33. Procédé selon l'une quelconque des revendications 24 à 32, dans lequel le site primaire est la capsule rénale, la prostate ou les testicules et/ou le site secondaire est choisi parmi les poumons, les reins, le foie, les ganglions lymphatiques, le cerveau, les os, les testicules, la rate, les ovaires et les glandes mammaires.

34. Procédé selon l'une quelconque des revendications 24 à 33, dans lequel l'amplification en chaîne par polymérase (PCR, polymerase chain reaction) par affichage différentiel est réalisée avec une amorce d'ancrage et une amorce variable.

35. Procédé selon la revendication 34, dans lequel l'amorce d'ancrage comprend une séquence de polythymidine et une séquence de dinucléotide connectée à un terminus-3'.

36. Procédé selon la revendication 34 ou la revendication 35, dans lequel l'amorce d'ancrage comprend une séquence de polythymidine qui comprend entre environ 5 et environ 30 thymidines et optionnellement la séquence de dinucléotide est choisie parmi AA, AG, AC, AT, GA, GG, GC, GT, CA, CG, CC et CT.

37. Procédé selon l'une quelconque des revendications 34 à 36, dans lequel l'amorce d'ancrage ou l'amorce variable comprend un groupe fonctionnel détectable choisi parmi les groupes fonctionnels radioactifs, les groupes fonctionnels phosphorescents, les groupes fonctionnels magnétiques, les groupes fonctionnels luminescents et les groupes fonctionnels conjugables.

38. Procédé selon l'une quelconque des revendications 34 à 37, dans lequel l'amorce d'ancrage et l'amorce variable ont une séquence commune.

39. Procédé selon l'une quelconque des revendications 24 à 38, dans lequel les cellules de mammifère sont dérivées d'un animal transgénique.
